# EUROPEAN PATENT APPLICATION

(11) **EP 4 406 487 A1**
(43) Date of publication of application: **31.07.2024**
(21) Application number: 22872647.7
(22) Date of filing: 29.08.2022
(51) Int. Cl.: A61B 8/12, A61B 1/00

(54) **ULTRASONIC ENDOSCOPE SYSTEM AND ULTRASONIC ENDOSCOPE SYSTEM OPERATING METHOD**

(30) Priority: 21.09.2021 JP 2021153270
(71) Applicant: FUJIFILM Corporation, Tokyo 106-8620 (JP)
(72) Inventor: YAMAMOTO, Katsuya, Ashigarakami-gun, Kanagawa 258-8538 (JP); MORIMOTO, Yasuhiko, Ashigarakami-gun, Kanagawa 258-8538 (JP)
(74) Representative: Hughes, Andrea Michelle
(86) International application number: PCT/JP2022/032373
(87) International publication number: WO 2023/047891

(57) **Abstract**

Provided is an ultrasound endoscope system including an ultrasound endoscope, in which a control circuit controls a transmission circuit such that a transmission of an ultrasound generating transmission signal for generating an ultrasound image and a transmission of a polarization process transmission signal for performing a polarization process are simultaneously performed on a plurality of ultrasound transducers on one side and the plurality of ultrasound transducers on the other side different from each other, and simultaneously performs transmission and reception of an ultrasonic wave and a polarization process on the plurality of ultrasound transducers different from each other.

Accordingly, it is possible to provide an ultrasound endoscope system capable of performing a polarization process on the plurality of ultrasound transducers on the other side, which pause a transmission of ultrasonic waves for acquiring an ultrasound image, by using an existing transmission circuit, simultaneously with a case where the transmission of the ultrasonic wave and a reception of a reflected wave are performed using the plurality of ultrasound transducers on one side to acquire the ultrasound image, and an operation method of an ultrasound endoscope system.

## Description

### BACKGROUND OF THE INVENTION

### 1. Field of the Invention

The present invention relates to an ultrasound endoscope system, which performs polarization process on a plurality of ultrasound transducers that an ultrasound endoscope comprises, and an operation method of an ultrasound endoscope system.

### 2. Description of the Related Art

As an ultrasound diagnostic apparatus for the purpose of biliary-pancreatic observation using the transgastrointestinal tract, an ultrasound endoscope in which an ultrasound observation portion is provided at the distal end portion of an endoscope is used. Such an ultrasound diagnostic apparatus acquires an ultrasound image of the inside of a body cavity of a subject by transmitting and receiving ultrasonic waves by driving a plurality of ultrasound transducers inside the body cavity of the subject. In the ultrasound diagnostic apparatus, it is necessary to avoid a reduction in sensitivity in a state in which the ultrasound diagnostic apparatus is placed inside the body cavity of the subject.

In the ultrasound diagnostic apparatus, the plurality of ultrasound transducers are, for example, single crystal transducers that are piezoelectric elements, and are usually used in a polarized state. The ultrasound transducer that is a single crystal transducer can receive ultrasonic waves with high sensitivity, but a depolarization phenomenon in which the degree of polarization decreases as the driving time increases may occur. In a case where a depolarization phenomenon occurs, the reception sensitivity of the ultrasound transducer decreases, which may affect the image quality of the ultrasound image. For this reason, as a measure against depolarization of the single crystal transducer, restoring the sensitivity by performing a repolarization process (hereinafter, also simply referred to as polarization process) is also known.

The risk of depolarization is correlated with the thickness of the transducer, that is, the resonance frequency of the transducer. The risk decreases as the thickness of the transducer increases (as the frequency of the transducer decreases). For this reason, the risk of depolarization is avoided by using a transducer using a single crystal transducer for body surface in a low frequency band of 1 to 6 MHz (refer to JP2013-005137A).

On the other hand, in the case of transmitting and receiving ultrasonic waves by driving each ultrasound transducer inside the body cavity of the subject, since it is necessary to set the frequency of the ultrasonic wave to a high frequency band of 7 to 8 MHz level, a transducer having a relatively small thickness is used. However, as the thickness of the transducer decreases, the risk of occurrence of a depolarization phenomenon increases. Therefore, in the case of a thin transducer, a process of repolarization is required (refer to JP2017-143353A and JP2020-000625A).

For this reason, techniques for countermeasures against depolarization in the ultrasound diagnostic apparatus have been developed so far. For example, an ultrasound sensor as a piezoelectric sensor apparatus described in JP2013-005137A has a piezoelectric element having a piezoelectric body and a pair of electrodes interposing the piezoelectric body therebetween, a detection circuit for performing detection process for detecting a detection signal output from the piezoelectric element, and a dedicated polarization process circuit for performing polarization process by applying a polarization voltage to the piezoelectric element. In the ultrasound sensor described in JP2013-005137A having such a configuration, it is possible to restore polarization by detecting depolarization from a difference in characteristics between the piezoelectric elements using the detection circuit and performing polarization process using the dedicated polarization process circuit. The polarization process is performed at a timing at which the electric power is supplied, a timing at which a request signal for performing detection process is input (each reception timing), or a timing at which a predetermined standby transition time has passed after the end of detection process, for example. Therefore, even in a case where a depolarization phenomenon occurs in the piezoelectric element, the piezoelectric element can be polarized again. As a result, it is possible to maintain the reception sensitivity of the piezoelectric element.

As another example, the ultrasound sensor described in JP2017-143353A has a piezoelectric element and a driving circuit for driving the piezoelectric element. The driving circuit drives the piezoelectric element with a driving waveform having, first, a step of maintaining the polarization of the piezoelectric element with a first potential V1, next, a step of transmitting an ultrasonic wave to the piezoelectric element by applying a maximum potential VH and a minimum potential VL at least once, next, a step of causing the piezoelectric element to stand by at a second potential V2, next, a step of increasing the second potential V2 to a third potential V3, next, a step of maintaining the third potential V3 while the piezoelectric element receives an ultrasonic wave, and next, a step of returning the third potential V3 to the first potential V1. In the ultrasound diagnostic apparatus described in JP2017-143353A having such a configuration, it is possible to drive the piezoelectric element while maintaining the polarization of the piezoelectric element by driving the piezoelectric element with the driving waveform having the above-described six steps. That is, JP2017-143353A describes that depolarization is prevented by studying the waveform for driving the piezoelectric element.

In addition, the ultrasound diagnostic apparatus described in JP2020-000625A has an ultrasound endoscope including an ultrasound observation portion, which transmits an ultrasonic wave to a subject and receives a reflected wave by using an ultrasound transducer, and an ultrasound processor device including a transmission circuit, which transmits a transmission signal to the ultrasound transducer to generate the ultrasonic wave, a reception circuit that outputs a reception signal based on the reflected wave, an ultrasound image generation unit that generates an ultrasound image based on the reception signal, and a control circuit that performs a polarization process on the ultrasound transducer using the transmission circuit in a non-diagnosis period in which the transmission of the ultrasonic wave and the reception of the reflected wave in a diagnosis period for acquiring the ultrasound image are not performed.

In the ultrasound diagnostic apparatus described in JP2020-000625A, polarization process of ultrasound transducers using an existing transmission circuit for transmitting a transmission signal to ultrasound transducers of an ultrasound endoscope, in a non-diagnosis time different from a time for acquiring an ultrasound image, without affecting the image quality of the ultrasound image and without causing a significant change in the circuit configuration and an increase in the circuit size.

### SUMMARY OF THE INVENTION

As described above, in the ultrasound sensor, the ultrasound apparatus, and the ultrasound diagnostic apparatus described in JP2013-005137A, JP2017-143353A, and JP2020-000625A, it is possible to restore or maintain the polarization of the ultrasound transducer and the piezoelectric element formed of a piezoelectric body.

However, as in the ultrasound sensor described in JP2013-005137A, providing a dedicated circuit for performing repolarization, a depolarization detection mechanism, and the like is a large hardware change factor. Accordingly, there is a problem in that it is very difficult to mount those described above in the existing system.

In the ultrasound sensor described in JP2017-143353A, in order to prevent depolarization and maintain polarization, the pulse length of the driving waveform is increased by inserting a DC component into each driving waveform. Accordingly, there is a problem in that the frame rate is reduced to affect the image quality of the ultrasound image. In addition, in order to prevent depolarization using such a driving waveform, there is a problem in that there is a tradeoff between the image quality and the risk of depolarization.

In addition, in the ultrasound diagnostic apparatus described in JP2020-000625A, in a case where a diagnosis period for acquiring the ultrasound image is long or frequently provided, even in a case where a repolarization process can be performed during the non-diagnosis period, as a result, a period in which the ultrasound transducer performs the transmission of ultrasonic wave and the reception reflected wave becomes longer, and depolarization of the ultrasound transducer progresses. Accordingly, there is a problem in that it is necessary to provide a period in which the transmission of ultrasonic wave and the reception reflected wave by the ultrasound transducer are stopped and repolarization of the ultrasound transducer is performed.

As in the ultrasound diagnostic apparatus described in JP2020-000625A, an ultrasound system to which an ultrasound endoscope is connected may be shared with body surface use. In this case, as for the specification of a simultaneous transmission opening number, 64 ch is often sufficient for an ultrasound endoscope having a small-diameter convex shape as compared with the specification for body surface (to 256 ch). For this reason, for the ultrasound endoscope, there is a problem in that the ultrasound system shared with body surface use is over-specified.

In addition, since a transmission waveform different from that in image visualization is usually used in the repolarization process, the ultrasound output is weak. However, in the ultrasound apparatus or the like in the related art, there is a problem in that the frame rate decreases in a case where the repolarization process is performed during scanning.

An object of the present invention is to solve the above-described problem of the related art and to provide an ultrasound endoscope system capable of performing a polarization process on the plurality of ultrasound transducers on the other side, which pause a transmission of ultrasonic waves for acquiring an ultrasound image but simultaneously transmit and open, by using an existing transmission circuit that transmits a transmission signal to an ultrasound transducer of an ultrasound endoscope, simultaneously with a case where the transmission of the ultrasonic wave and a reception of a reflected wave are performed using the plurality of ultrasound transducers on one side to acquire the ultrasound image, and an operation method of an ultrasound endoscope system.

To achieve the above object, an ultrasound endoscope system a first aspect of the present invention is an ultrasound endoscope system that acquires an ultrasound image and an endoscope image, the ultrasound endoscope system comprising an ultrasound endoscope including an ultrasound observation portion, which has an ultrasound transducer array in which a plurality of ultrasound transducers that simultaneously transmit and open are arranged, which transmits an ultrasonic wave using the plurality of ultrasound transducers on one side of the plurality of ultrasound transducers, receives a reflected wave of the ultrasonic wave due to the transmission of the ultrasonic wave, and pauses the transmission of the ultrasonic waves by the plurality of ultrasound transducers on the other side of the plurality of ultrasound transducers; and an ultrasound processor device including a transmission circuit, which transmits an ultrasound generating transmission signal consisting of diagnostic driving pulses to be applied to the plurality of ultrasound transducers on one side to generate the ultrasonic waves from the plurality of ultrasound transducers on one side and simultaneously transmits a polarization process transmission signal consisting of polarization driving pulse for performing a polarization process to the plurality of ultrasound transducers on the other side in which the transmission of the ultrasound generating transmission signal for acquiring the ultrasound image is paused, a reception circuit, which outputs a reception signal based on the reflected waves received by the plurality of ultrasound transducers on one side, and an ultrasound image generation unit, which generates the ultrasound image by imaging the reception signal to acquire the ultrasound image, in which the ultrasound processor device further includes a control circuit that transmits the ultrasound generating transmission signal to the plurality of ultrasound transducers on one side using the transmission circuit to cause the plurality of ultrasound transducers on one side to perform the transmission of the ultrasonic wave and the reception of the reflected wave, to acquire the ultrasound image, and that transmits the polarization process transmission signal to the plurality of ultrasound transducers on the other side using the transmission circuit to be subjected to the polarization process, and the control circuit controls the transmission circuit such that the transmission of the ultrasound generating transmission signal for generating the ultrasound image and the transmission of the polarization process transmission signal for performing the polarization process are simultaneously performed on the plurality of ultrasound transducers on one side and the plurality of ultrasound transducers on the other side different from each other, and simultaneously performs transmission and reception of the ultrasonic wave and the polarization process on the plurality of ultrasound transducers different from each other.

Here, it is preferable that a transmission waveform of the ultrasound generating transmission signal is different from a transmission waveform of the polarization process transmission signal.

In addition, it is preferable that at least one of a frequency, voltage, or wave number of the transmission waveform is different between the ultrasound generating transmission signal and the polarization process transmission signal.

In addition, it is preferable that the control circuit changes a simultaneous transmission opening number of the plurality of ultrasound transducers on the other side on which the polarization process is performed, in response to a change in a simultaneous transmission opening number of the plurality of ultrasound transducers on one side that perform the transmission and reception of the ultrasonic wave for generating the ultrasound image in accordance with a change in a focus position for observation.

In addition, it is preferable that in a case of using the ultrasound transducer array in which the plurality of ultrasound transducers are arranged in a circumferential shape, a center of the plurality of ultrasound transducers on one side that perform the transmission and reception of the ultrasonic wave for generating the ultrasound image and a center of the plurality of ultrasound transducers on the other side that perform the transmission of the polarization process transmission signal for performing the polarization process are arranged at positions different from each other by 180 degrees.

In addition, it is preferable that the control circuit controls, in a case where the transmission of the ultrasonic wave and the reception of the reflected wave are performed using the plurality of ultrasound transducers on one side, simultaneously, the plurality of ultrasound transducers on the other side to pause the transmission and reception of the ultrasonic wave and to be subjected to the polarization process, and conversely, in a case where the transmission of the ultrasonic wave and the reception of the reflected wave are performed using the plurality of ultrasound transducers on the other side, simultaneously, the plurality of ultrasound transducers on one side to pause the transmission and reception of the ultrasonic wave and to be subjected to the polarization process.

In addition, to achieve the above object, an operation method of an ultrasound endoscope system a second aspect of the present invention is an operation method of an ultrasound endoscope system that acquires an ultrasound image and an endoscope image, the ultrasound endoscope system including an ultrasound endoscope including an ultrasound observation portion, which has an ultrasound transducer array in which a plurality of ultrasound transducers are arranged, and an ultrasound processor device including a transmission circuit, which transmits an ultrasound generating transmission signal to the plurality of ultrasound transducers on one side of the plurality of ultrasound transducers and simultaneously transmits a polarization process transmission signal to the plurality of ultrasound transducers on the other side in which the transmission of the ultrasound generating transmission signal for acquiring the ultrasound image is paused, a reception circuit, which outputs a reception signal based on reflected waves received by the plurality of ultrasound transducers on one side, and an ultrasound image generation unit, which generates the ultrasound image by imaging the reception signal, the operation method comprising a control step of controlling the transmission circuit to generate the ultrasound generating transmission signal consisting of diagnostic driving pulses to be applied to the plurality of ultrasound transducers on one side, which generate ultrasonic waves for acquiring the ultrasound image, and, for a polarization process, to generate the polarization process transmission signal consisting of polarization driving pulses applied to the plurality of ultrasound transducers on the other side in which the transmission of the ultrasound generating transmission signal is paused; a generating step of transmitting the ultrasound generating transmission signal generated from the transmission circuit to the plurality of ultrasound transducers on one side, and applying the diagnostic driving pulses to the plurality of ultrasound transducers on one side to generate the ultrasonic waves; a reception step of receiving the reflected waves of the ultrasonic waves via the plurality of ultrasound transducers on one side; an output step of outputting the reception signal based on the reflected waves received by the plurality of ultrasound transducers, from the reception circuit; a generation step of generating the ultrasound image via the ultrasound image generation unit receiving the reception signal and imaging the received reception signal; and a polarization step of transmitting the polarization process transmission signal generated from the transmission circuit to the plurality of ultrasound transducers on the other side and applying the polarization driving pulses to the plurality of ultrasound transducers on the other side to perform the polarization process on the plurality of ultrasound transducers on the other side simultaneously while executing the generating step and the reception step.

Here, it is preferable that a transmission waveform of the ultrasound generating transmission signal is different from a transmission waveform of the polarization process transmission signal.

In addition, it is preferable that at least one of a frequency, voltage, or wave number of the transmission waveform is different between the ultrasound generating transmission signal and the polarization process transmission signal.

In addition, it is preferable that a simultaneous transmission opening number of the plurality of ultrasound transducers on the other side on which the polarization process is performed is changed, in response to a change in a simultaneous transmission opening number of the plurality of ultrasound transducers on one side that perform transmission and reception of the ultrasonic wave for generating the ultrasound image in accordance with a change in a focus position for observation.

In addition, it is preferable that in a case of using the ultrasound transducer array in which the plurality of ultrasound transducers are arranged in a circumferential shape, a center of the plurality of ultrasound transducers on one side that perform transmission and reception of the ultrasonic wave for generating the ultrasound image and a center of the plurality of ultrasound transducers on the other side that perform the transmission of the polarization process transmission signal for performing the polarization process are arranged at positions different from each other by 180 degrees.

Further, it is preferable that in the control step, in a case of being controlled to generate the ultrasound generating transmission signal to be transmitted to the plurality of ultrasound transducers on one side, a control is performed to generate the polarization process transmission signal to be transmitted to the plurality of ultrasound transducers on the other side, the generating step and the reception step are performed on the plurality of ultrasound transducers on one side, and the polarization step is simultaneously performed on the plurality of ultrasound transducers on the other side, conversely, in the control step, in a case of being controlled to generate the ultrasound generating transmission signal to be transmitted to the plurality of ultrasound transducers on the other side, a control is performed to generate the polarization process transmission signal to be transmitted to the plurality of ultrasound transducers on one side, and the generating step and the reception step are performed on the plurality of ultrasound transducers on the other side, and the polarization step is simultaneously performed on the plurality of ultrasound transducers on one side.

According to the present invention, in a case where a transmission of an ultrasonic wave and a reception of a reflected wave are performed using the plurality of ultrasound transducers on one side to acquire an ultrasound image, simultaneously, using an existing transmission circuit that transmits a transmission signal to an ultrasound transducer of an ultrasound endoscope, it is possible to perform a polarization process on the plurality of ultrasound transducers on the other side that pause the transmission of the ultrasonic waves for acquiring the ultrasound image but simultaneously transmit and open.

Therefore, according to the present invention, without degrading the image quality of the ultrasound image acquired by using the plurality of ultrasound transducers on one side, simultaneously, the polarization process of the plurality of ultrasound transducers on the other side can be performed, and a polarization state of the ultrasound transducer can always be kept satisfactory.

Therefore, according to the present invention, since the reception sensitivities of the plurality of ultrasound transducers can always be kept satisfactory without reducing the image quality of the ultrasound image, a high-quality ultrasound image can always be acquired without affecting the image quality of the ultrasound image.

In addition, according to the present invention, the polarization process of a plurality of ultrasound transducers is performed using the existing transmission circuit for transmitting the transmission signal to the ultrasound transducers of the ultrasound endoscope. Therefore, it is possible to perform the polarization process of the ultrasound transducers without significantly changing the existing circuit configuration and without causing an increase in the circuit size.

In addition, according to the present invention, since the polarization process is performed on the ultrasound transducer that pauses the transmission of the ultrasonic wave for acquiring the ultrasound image, a frame rate for acquiring the ultrasound image is not reduced.

Moreover, according to the present invention, even in an ultrasound system in which an ultrasound endoscope having a small number of channels (for example, 64 ch) of a simultaneous transmission opening number and an ultrasound probe (probe) for body surface having a large number of channels (for example, 256 ch) are shared, it is possible to perform the polarization process on the ultrasound transducer that pauses the transmission of the ultrasonic wave for acquiring the ultrasound image, and thus the ultrasound system is not over-specified.

### BRIEF DESCRIPTION OF THE DRAWINGS

Fig. 1 is a diagram showing the schematic configuration of an ultrasound endoscope system according to an embodiment of the present invention.
Fig. 2 is a plan view showing a distal end portion of an insertion part of an ultrasound endoscope and its periphery shown in Fig. 1.
Fig. 3 is a diagram showing a cross section of the distal end portion of the insertion part of the ultrasound endoscope shown in Fig. 2 taken along line I-I in Fig. 2.
Fig. 4 is a block diagram showing the configuration of an ultrasound processor device shown in Fig. 1.
Fig. 5A is a graph showing an example of a driving waveform of a polarization driving pulse transmitted from a transmission circuit shown in Fig. 4.
Fig. 5B is a graph showing the relationship between the frequency and the sensitivity of the driving waveform of the polarization driving pulse shown in Fig. 5A.
Fig. 6A is a graph showing another example of the driving waveform of the polarization driving pulse transmitted from the transmission circuit shown in Fig. 4.
Fig. 6B is a graph showing the relationship between the frequency and the sensitivity of the driving waveform of the polarization driving pulse shown in Fig. 5A and the relationship between the frequency and the sensitivity of the driving waveform of the polarization driving pulse shown in Fig. 6A.
Fig. 7A is a graph showing another example of the pulse waveform of the polarization driving pulse transmitted from the transmission circuit shown in Fig. 4.
Fig. 7B is a graph showing the relationship between the frequency and the sensitivity of the driving waveform of the polarization driving pulse shown in Fig. 7A.
Fig. 7C is a graph showing another example of the pulse waveform of the polarization driving pulse transmitted from the transmission circuit shown in Fig. 4.
Fig. 7D is a graph showing the relationship between the frequency and the sensitivity of the driving waveform of the polarization driving pulse shown in Fig. 7C.
Fig. 8A is a graph showing another example of the pulse waveform of the diagnostic driving pulse transmitted from the transmission circuit shown in Fig. 4.
Fig. 8B is a graph showing the relationship between the frequency and the sensitivity of the driving waveform of the diagnostic driving pulse shown in Fig. 8A.
Fig. 9 is a diagram showing a configuration of an ultrasound system in which the number of channels of a pulse generating circuit is smaller than the number of channels of an ultrasound transducer.
Fig. 10 is a diagram showing a configuration of a microconvex transducer.
Fig. 11A is a diagram showing an example of a state of a plurality of ultrasound transducers driven for image formation and a plurality of ultrasound transducers to be subjected to a polarization process simultaneously with driving of the ultrasound transducers.
Fig. 11B is a diagram showing another example of the state of the plurality of ultrasound transducers driven for the image formation and the plurality of ultrasound transducers simultaneously to be subjected to the polarization process with driving of the ultrasound transducers.
Fig. 11C is a diagram showing another example of the state of the plurality of ultrasound transducers driven for the image formation and the plurality of ultrasound transducers simultaneously to be subjected to the polarization process with driving of the ultrasound transducers.
Fig. 12A is a diagram showing an example of a state in which in a radial transducer array, a center of the plurality of ultrasound transducers for the image formation and a center of the plurality of ultrasound transducers for the polarization process are arranged at positions different from each other by 180 degrees.
Fig. 12B is a diagram showing an example of a state in which in the radial transducer array, a center of the plurality of ultrasound transducers for the image formation and a center of the plurality of ultrasound transducers for the polarization process are arranged at positions different from each other by 180 degrees.
Fig. 13 is a flowchart showing the flow of the diagnosis process using the ultrasound endoscope system shown in Fig. 1.
Fig. 14 is a flowchart showing the procedure of a diagnosis step in the diagnosis process.
Fig. 15 is a conceptual diagram of an example showing display modes.

### DESCRIPTION OF THE PREFERRED EMBODIMENTS

An ultrasound endoscope system according to an embodiment of the present invention will be described in detail below based on preferred embodiments shown in the accompanying diagrams.

The present embodiment is a representative embodiment of the present invention, but is merely an example and does not limit the present invention.

In addition, in the present specification, a numerical range represented using "to" means a range including numerical values described before and after the preposition "to" as a lower limit value and an upper limit value.

### «Outline of Ultrasound Endoscope System»

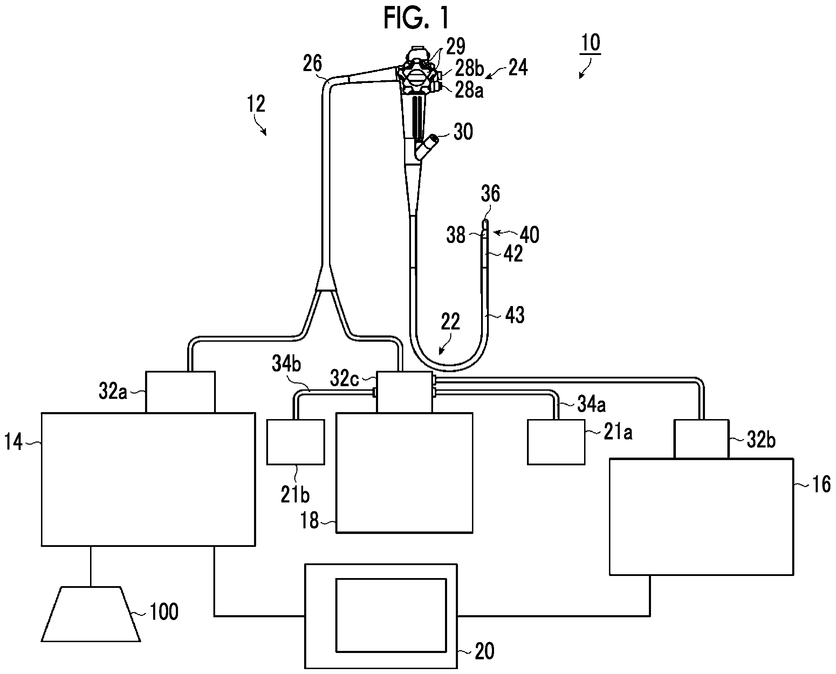

The outline of an ultrasound endoscope system 10 according to the present embodiment will be described with reference to Fig. 1. Fig. 1 is a diagram showing the schematic configuration of the ultrasound endoscope system 10.

The ultrasound endoscope system 10 is used to make observation (hereinafter, also referred to as ultrasound diagnosis) a state of an observation target part in a body of a patient as a subject using ultrasonic waves. Here, the observation target part is a part that is difficult to examine from the body surface side of the patient, for example, a gallbladder or a pancreas. With the use of the ultrasound endoscope system 10, it is possible to perform ultrasound diagnosis of a state of the observation target part and the presence or absence of an abnormality by way of digestive tracts, such as esophagus, stomach, duodenum, small intestine, and large intestine, which are body cavities of the patient.

The ultrasound endoscope system 10 acquires an ultrasound image and an endoscope image, and as shown in Fig. 1, has the ultrasound endoscope 12, an ultrasound processor device 14, the endoscope processor device 16, the light source device 18, a monitor 20, a water supply tank 21a, a suction pump 21b, and a console 100.

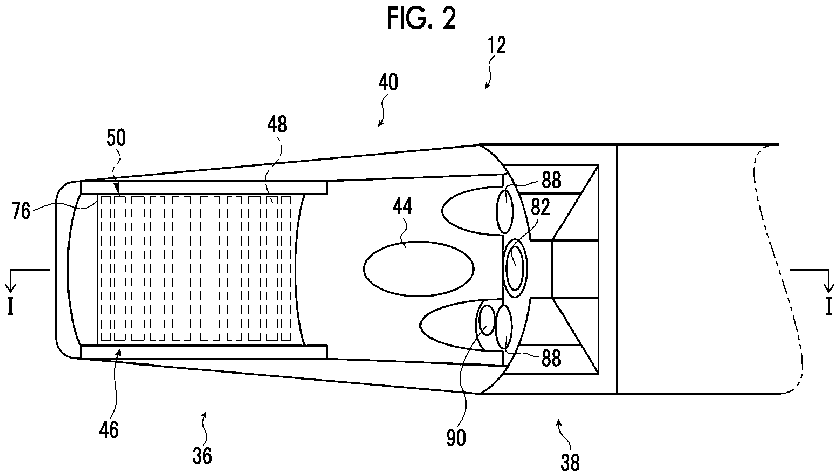

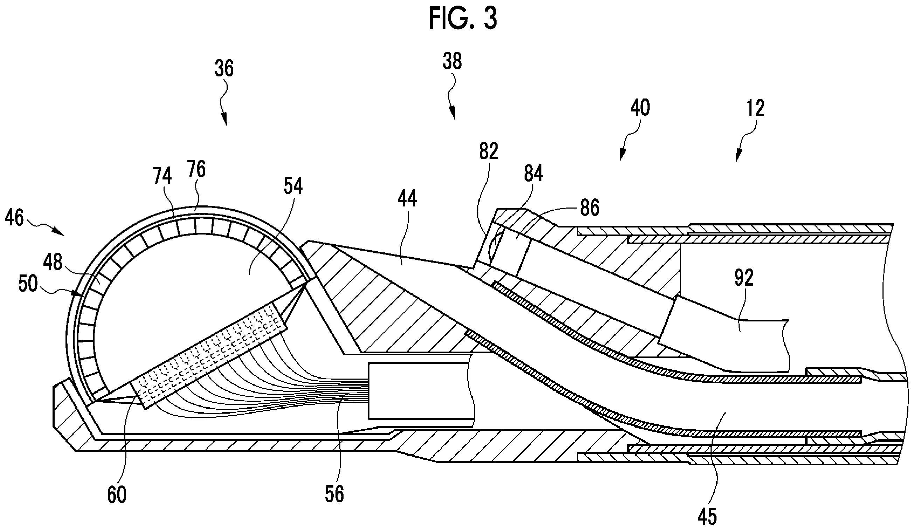

The ultrasound endoscope 12 is an endoscope, and comprises an insertion part 22 to be inserted into the body cavity of a patient, an operating part 24 operated by an operator (user), such as a doctor or a technician, and an ultrasound transducer unit 46 attached to a distal end portion 40 of the insertion part 22 (refer to Figs. 2 and 3). By the function of the ultrasound endoscope 12, the operator can acquire an endoscope image of a body cavity inner wall of the patient and an ultrasound image of the observation target part.

Here, the "endoscope image" is an image that is obtained by imaging the body cavity inner wall of the patient using an optical method. Furthermore, the "ultrasound image" is an image that is obtained by receiving reflected waves (echoes) of ultrasonic waves transmitted from the inside of the body cavity of the patient toward the observation target part and imaging reception signals.

The ultrasound endoscope 12 will be described below in detail.

The ultrasound processor device 14 is connected to the ultrasound endoscope 12 through a universal cord 26 and an ultrasound connector 32a provided at an end portion of the universal cord 26. The ultrasound processor device 14 controls the ultrasound transducer unit 46 of the ultrasound endoscope 12 to transmit the ultrasonic wave. In addition, the ultrasound processor device 14 generates an ultrasound image by imaging the reception signal in a case where the reflected wave (echo) of the transmitted ultrasonic wave is received by the ultrasound transducer unit 46.

The ultrasound processor device 14 will be described in detail later.

The endoscope processor device 16 is connected to the ultrasound endoscope 12 through the universal cord 26 and an endoscope connector 32b provided at an end portion of the universal cord 26. The endoscope processor device 16 generates an endoscope image by acquiring image data of an observation target adjacent part imaged by the ultrasound endoscope 12 (more specifically, a solid-state imaging element 86 to be described later, see Fig. 3) and performing predetermined image processing on the acquired image data.

Here, the "observation target adjacent part" is a portion that is at a position adjacent to the observation target part in the body cavity inner wall of the patient.

In the present embodiment, the ultrasound processor device 14 and the endoscope processor device 16 are formed by two devices (computers) provided separately. However, the present invention is not limited thereto, and both the ultrasound processor device 14 and the endoscope processor device 16 may be formed by one device.

The light source device 18 is connected to the ultrasound endoscope 12 through the universal cord 26 and a light source connector 32c provided at the end portion of the universal cord 26. The light source device 18 emits white light or specific wavelength light formed of three primary color light components of red light, green light, and blue light in a case of imaging the observation target adjacent part using the ultrasound endoscope 12. The light emitted from the light source device 18 propagates through the ultrasound endoscope 12 through a light guide (not shown) included in the universal cord 26, and is emitted from the ultrasound endoscope 12 (more specifically, an illumination window 88 to be described later, see Fig. 2). As a result, the observation target adjacent part is illuminated with the light from the light source device 18.

The monitor 20 is connected to the ultrasound processor device 14 and the endoscope processor device 16, and displays an ultrasound image generated by the ultrasound processor device 14 and an endoscope image generated by the endoscope processor device 16. As a display method of the ultrasound image and the endoscope image, either one of the images may be switched and displayed on the monitor 20, or both the images may be displayed at the same time. Display modes of the ultrasound image and the endoscope image will be described later.

In the present embodiment, although the ultrasound image and the endoscope image are displayed on one monitor 20, a monitor for ultrasound image display and a monitor for endoscope image display may be provided separately. In addition, the ultrasound image and the endoscope image may be displayed in a display form other than the monitor 20. For example, the ultrasound image and the endoscope image may be displayed on a display of a terminal carried by the operator.

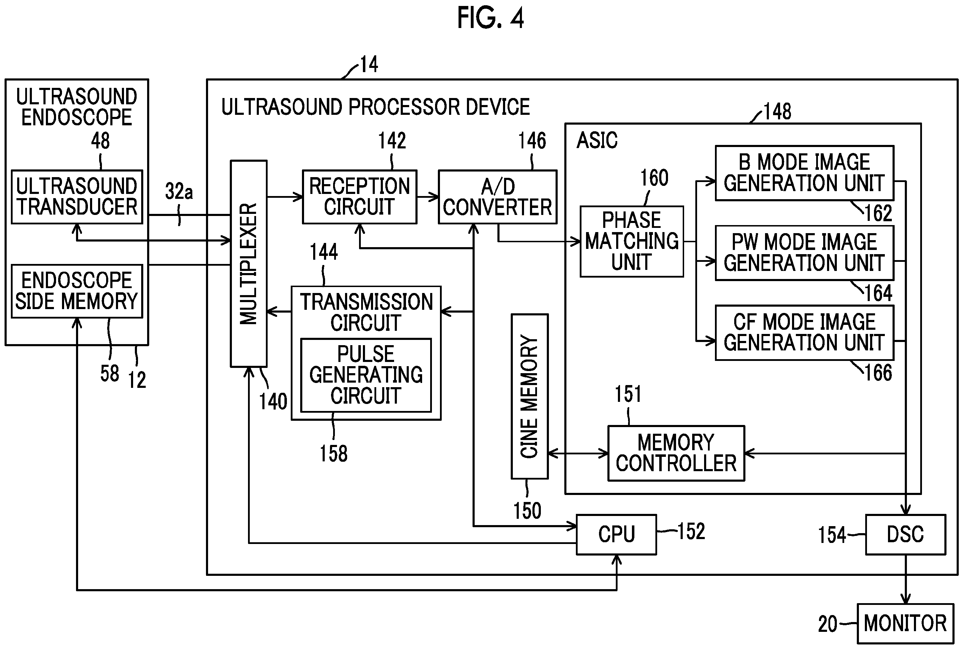

The console 100 is a device provided for the operator to input information necessary for ultrasound diagnosis or for the operator to instruct the ultrasound processor device 14 to start ultrasound diagnosis. The console 100 is configured to include, for example, a keyboard, a mouse, a trackball, a touch pad, and a touch panel. In a case where the console 100 is operated, a CPU (control circuit) 152 (refer to Fig. 4) of the ultrasound processor device 14 controls each unit of the device (for example, a reception circuit 142 and a transmission circuit 144 to be described later) according to the operation content.

Specifically, the operator inputs examination information (for example, examination order information including date, an order number, and the like and patient information including a patient ID, a patient name, and the like) through the console 100 in a state before starting ultrasound diagnosis. In a case where the operator gives an instruction to start the ultrasound diagnosis through the console 100 after the input of the examination information is completed, the CPU 152 of the ultrasound processor device 14 controls each unit of the ultrasound processor device 14 so that the ultrasound diagnosis is performed based on the input examination information.

Furthermore, the operator can set various control parameters through the console 100 in executing ultrasound diagnosis. As the control parameters, for example, a selection result of a live mode and a freeze mode, a set value of a display depth (depth), a selection result of an ultrasound image generation mode, and the like are exemplified.

Here, the "live mode" is a mode where ultrasound images (moving image) obtained at a predetermined frame rate are displayed successively (displayed in real time). The "freeze mode" is a mode in which an image (still image) of one frame of an ultrasound image (moving image) generated in the past is read out from a cine memory 150 to be described later and displayed.

There are a plurality of ultrasound image generation modes that can be selected in the present embodiment. Specifically, there are a brightness (B) mode, a color flow (CF) mode, and a pulse wave (PW) mode. The B mode is a mode in which a tomographic image is displayed by converting the amplitude of the ultrasound echo into a brightness. The CF mode is a mode where an average blood flow speed, flow fluctuation, intensity of a flow signal, flow power, or the like are mapped to various colors and superimposedly displayed on a B mode image. The PW mode is a mode in which the speed (for example, speed of blood flow) of the ultrasound echo source detected based on the transmission and reception of the pulse wave is displayed.

The above-described ultrasound image generation modes are merely an example, and modes other than the above-described three kinds of modes, for example, an amplitude (A) mode, a motion (M) mode, and a contrast mode may be further included or a mode in which a Doppler image is obtained may be included.

### «Configuration of Ultrasound Endoscope 12»

Next, the configuration of the ultrasound endoscope 12 will be described with reference to Fig. 1 described above, and Figs. 2 to 4. Fig. 2 is an enlarged plan view showing a distal end portion of an insertion part 22 of an ultrasound endoscope 12 and the periphery thereof. Fig. 3 is a cross-sectional view showing a cross section of the distal end portion 40 of the insertion part 22 of the ultrasound endoscope 12 taken along line I-I in Fig. 2.

As described above, the ultrasound endoscope 12 has the insertion part 22 and the operating part 24. As shown in Fig. 1, the insertion part 22 comprises the distal end portion 40, a bendable portion 42, and a soft portion 43 in order from the distal end side (free end side). As shown in Fig. 2, the ultrasound observation portion 36 and the endoscope observation portion 38 are provided in the distal end portion 40. As shown in Fig. 3, the ultrasound transducer unit 46 comprising a plurality of ultrasound transducers 48 is arranged in the ultrasound observation portion 36.

Furthermore, as shown in Fig. 2, a treatment tool outlet port 44 is provided in the distal end portion 40. The treatment tool outlet port 44 serves as an outlet of a treatment tool (not shown), such as forceps, an puncture needle, or a high frequency scalpel. In addition, the treatment tool outlet port 44 serves as a suction port in the case of sucking aspirates, such as blood and body waste.

The bendable portion 42 is a portion consecutively provided on a proximal end side (a side opposite to a side on which the ultrasound transducer unit 46 is provided) than the distal end portion 40, and is freely bent. The soft portion 43 is a portion connecting the bendable portion 42 and the operating part 24 to each other, has flexibility, and is provided so as to extend in an elongated state.

A plurality of pipe lines for air and water supply and a plurality of pipe lines for suction are formed inside each of the insertion part 22 and the operating part 24. In addition, a treatment tool channel 45 whose one end communicates with the treatment tool outlet port 44 is formed in each of the insertion part 22 and the operating part 24.

Next, the ultrasound observation portion 36, the endoscope observation portion 38, the water supply tank 21a, the suction pump 21b, and the operating part 24 among the components of the ultrasound endoscope 12 will be described in detail.

### (Ultrasound Observation Portion)

The ultrasound observation portion 36 is a portion that is provided to acquire an ultrasound image, and is arranged on the distal end side in the distal end portion 40 of the insertion part 22. As shown in Fig. 3, the ultrasound observation portion 36 comprises the ultrasound transducer unit 46, a plurality of coaxial cables 56, and a flexible printed circuit (FPC) 60.

The ultrasound transducer unit 46 corresponds to an ultrasound probe (probe), transmits ultrasonic waves using an ultrasound transducer array 50, in which a plurality of ultrasound transducers 48 described below are arranged, inside a body cavity of a patient, receives reflected waves (echoes) of the ultrasonic waves reflected by the observation target part, and outputs reception signals. The ultrasound transducer unit 46 according to the present embodiment is a convex type, and transmits an ultrasonic wave radially (in an arc shape). However, the type (model) of the ultrasound transducer unit 46 is not particularly limited, and other types may be used as long as it is possible to transmit and receive ultrasonic waves. For example, a sector type, a linear type, and a radial type may be used.

As shown in Fig. 3, the ultrasound transducer unit 46 is formed by laminating a backing material layer 54, the ultrasound transducer array 50, an acoustic matching layer 74, and an acoustic lens 76.

The ultrasound transducer array 50 has a plurality of ultrasound transducers 48 arranged in a one-dimensional array. More specifically, the ultrasound transducer array 50 is formed by arranging N (for example, N = 64, 128, or 256) ultrasound transducers 48 at equal intervals in a convex bending shape along the axial direction of the distal end portion 40 (longitudinal axis direction of the insertion part 22). The ultrasound transducer array 50 may be one in which a plurality of ultrasound transducers 48 are arranged in a two-dimensional array.

Each of the N ultrasound transducers 48 is formed by arranging electrodes on both surfaces of a single crystal transducer that is a piezoelectric element. As the single crystal transducer, any of quartz, lithium niobate, lead magnesium niobate (PMN), lead magnesium niobate-lead titanate (PMN-PT), lead zinc niobate (PZN), lead zinc niobate-lead titanate (PZN-PT), lead indium niobate (PIN), lead titanate (PT), lithium tantalate, langasite, and zinc oxide can be used.

The electrodes have an individual electrode (not shown) individually provided for each of a plurality of ultrasound transducers 48 and a transducer ground (not shown) common to a plurality of ultrasound transducers 48. In addition, the electrodes are electrically connected to the ultrasound processor device 14 through the coaxial cable 56 and the FPC 60.

The ultrasound transducer 48 according to the present embodiment needs to be driven (vibrated) at a relatively high frequency of 7 MHz to 8 MHz level in order to acquire an ultrasound image in the body cavity of the patient. For this reason, the thickness of the piezoelectric element forming the ultrasound transducer 48 is designed to be relatively small. For example, the thickness of the piezoelectric element forming the ultrasound transducer 48 is 75 µm to 125 µm, preferably 90 µm to 110 µm.

A diagnostic driving pulse that has a pulsed driving voltage is supplied from the ultrasound processor device 14 to each ultrasound transducer 48, as an input signal (transmission signal), through the coaxial cable 56. In a case where the driving voltage is applied to the electrodes of the ultrasound transducer 48, the piezoelectric element expands and contracts to drive (vibrate) the ultrasound transducer 48. As a result, a pulsed ultrasonic wave is output from the ultrasound transducer 48. In this case, the amplitude of the ultrasonic wave output from the ultrasound transducer 48 has magnitude according to intensity (output intensity) in a case where the ultrasound transducer 48 outputs the ultrasonic wave. Here, the output intensity is defined as the magnitude of the sound pressure of the ultrasonic wave output from the ultrasound transducer 48.

Each ultrasound transducer 48 vibrates (is driven) with reception of a reflected wave (echo) of the ultrasonic wave, and the piezoelectric element of each ultrasound transducer 48 generates an electric signal. The electric signal is output from each ultrasound transducer 48 to the ultrasound processor device 14 as a reception signal of the ultrasonic wave. In this case, the magnitude (voltage value) of the electric signal output from the ultrasound transducer 48 has a magnitude corresponding to the reception sensitivity in a case where the ultrasound transducer 48 receives the ultrasonic wave. Here, the reception sensitivity is defined as a ratio of the amplitude of the electric signal, which is output from the ultrasound transducer 48 in response to reception of the ultrasonic wave, to the amplitude of the ultrasonic wave transmitted by the ultrasound transducer 48.

In the present embodiment, the N ultrasound transducers 48 are driven sequentially by an electronic switch, such as a multiplexer 140 (see Fig. 4), scanning with ultrasonic waves is performed in a scanning range along a curved surface on which the ultrasound transducer array 50 is arranged, for example, a range of about several tens of mm from the center of curvature of the curved surface, and the ultrasound transducer array 50 that is not scanned is subjected to a polarization process. More specifically, in a case where the maximum simultaneous opening number of the transmission circuit 144 (the maximum number of channels of the pulser (pulse generating circuit) 158) is M (for example, 64), and the transmission circuit 144 is connected to the N (M ≤ N, for example, 128) ultrasound transducers 48, a multiplexer 140 of M:N (for example, 1:2) is used. For example, in a case where M is 64 and N is 128, the No. 1 circuit of the transmission circuit 144 is connected to the No. 1 and No. 65 ultrasound transducers 48, the No. 2 circuit of the transmission circuit 144 is connected to the No. 2 and No. 66 ultrasound transducers 48, and subsequently, the No. p (p = 3 to 63) circuit of the transmission circuit 144 is connected to the No. p and No. (p + 64) ultrasound transducers 48 similarly, and the last No. 64 circuit of the transmission circuit 144 is connected to the No. 64 and No. 128 ultrasound transducers 48. Accordingly, the No. 1 and No. 65 ultrasound transducers 48 are connected to the same No. 1 channel of the pulser 158 of the transmission circuit 144 and thus cannot be simultaneously driven. Similarly, the No. 2 and No. 66 ultrasound transducers 48, and subsequently, the No. p and No. (p + 64) ultrasound transducers 48, and finally the No. 64 and No. 128 ultrasound transducers 48 cannot also be simultaneously driven.

Here, in a case where the m (m < M, for example, M/2 (32 elements)) ultrasound transducers 48 are driven, the (M - m) channels of pulser 158 are left, and the (M - m) ultrasound transducers 48 are not used for driving. Accordingly, in the present invention, the polarization process is performed on the (M - m) ultrasound transducers 48 that are not driven by using the left (M - m) channels of the pulser 158.

For example, in a case where the 32 Nos. 1 to 32 ultrasound transducers 48 are driven to acquire an ultrasound image, that is, to visualize an image, the 32 Nos. 65 to 96 ultrasound transducers 48 are connected to the same pulser 158 and thus cannot be driven. Meanwhile, either the 32 Nos. 33 to 64 ultrasound transducers 48 or the 32 Nos. 97 to 128 ultrasound transducers 48 other than these ultrasound transducers can be selected by the multiplexer 140 for the polarization process.

Accordingly, while the 32 Nos. 1 to 32 ultrasound transducers 48 selected by the multiplexer 140 are driven for image visualization, the 32 Nos. 33 to 64 or Nos. 97 to 128 ultrasound transducers 48 selected by the multiplexer 140 can be subjected to the polarization process.

For example, in the case of acquiring a B mode image (tomographic image) as an ultrasound image, a driving voltage is supplied to m (for example, m = M/2) ultrasound transducers 48 (hereinafter, also referred to as driving target transducers) arranged in series, among the N ultrasound transducers 48, by opening channel selection of the multiplexer 140. With this, the m driving target transducers are driven, and an ultrasonic wave is output from each driving target transducer of the opening channel. The ultrasonic waves output from the m driving target transducers are immediately composed, and the composite wave (ultrasound beam) is transmitted toward the observation target part. Thereafter, each of the m driving target transducers receives an ultrasonic wave (echo) reflected at the observation target part, and outputs an electric signal (reception signal) corresponding to the reception sensitivity at that point in time.

In addition, in the present embodiment, simultaneously with driving of m ultrasound transducers 48 described above, by opening channel selection for polarization by the multiplexer 140, the polarization voltage different from the driving voltage of the driving target transducer 48 is supplied to, for example, (M - m) ultrasound transducers 48 (hereinafter, also referred to as polarization target transducer) among N ultrasound transducers 48 that are not used for acquiring the ultrasound image, to perform the polarization process. The polarization process of the ultrasound transducer 48 will be described below in detail.

Meanwhile, the above-described series of image visualization steps for acquiring the ultrasound image (that is, the supply of the driving voltage to the driving target transducer, the transmission and reception of the ultrasonic waves, and the output of the electric signal) may be repeatedly performed while shifting the positions of the driving target transducers among the N ultrasound transducers 48 one by one (one ultrasound transducer 48 at a time). In correspondence with this, the above-described series of polarization process steps for the polarization process may also be repeatedly performed while shifting the positions of the polarization target transducers among the N ultrasound transducers 48 one by one (one ultrasound transducer 48 at a time).

In this case, specifically, the above-described series of image visualization steps are started from m driving target transducers on both sides of the ultrasound transducer 48 located at one end among the N ultrasound transducers 48. The above-described series of polarization process steps are also started from (M - m) polarization target transducers corresponding to the m driving target transducers. Then, the above-described series of image visualization steps are repeated each time the position of the driving target transducer is shifted due to switching of the opening channel by the multiplexer 140. Similarly, the above-described series of polarization process steps are also repeated each time the position of the polarization target transducer corresponding to the driving target transducer is shifted due to switching of the opening channel by the multiplexer 140. Finally, the above-described series of image visualization steps are repeatedly performed a total of N times up to m driving target transducers on both sides of the ultrasound transducer 48 located at the other end among the N ultrasound transducers 48. Meanwhile, the above-described series of polarization process steps are also repeatedly performed a total of N times up to (M - m) polarization target transducers corresponding to m driving target transducers on both sides of the ultrasound transducer 48 located at the other end. In this manner, for the acquisition of the ultrasound image, the N ultrasound transducers 48 can be uniformly used, and the N ultrasound transducers 48 can be uniformly subjected to the polarization process.

The backing material layer 54 supports each ultrasound transducer 48 of the ultrasound transducer array 50 from a rear surface side. Furthermore, the backing material layer 54 has a function of attenuating ultrasonic waves propagating to the backing material layer 54 side among ultrasonic waves emitted from the ultrasound transducers 48 or ultrasonic waves (echoes) reflected by the observation target part. The backing material is a material having rigidity, such as hard rubber, and an ultrasound attenuating material (ferrite, ceramics, and the like) is added as necessary.

The acoustic matching layer 74 is superimposed on the ultrasound transducer array 50, and is provided for acoustic impedance matching between the body of the patient and the ultrasound transducer 48. The acoustic matching layer 74 is provided, whereby it is possible to increase the transmittance of the ultrasonic wave. As a material of the acoustic matching layer 74, it is possible to use various organic materials whose acoustic impedance values are closer to that of the body of the patient than the piezoelectric element of the ultrasound transducer 48. Specific examples of the material of the acoustic matching layer 74 include epoxy resin, silicone rubber, polyimide, polyethylene, and the like.

The acoustic lens 76 superimposed on the acoustic matching layer 74 converges ultrasonic waves emitted from the ultrasound transducer array 50 toward the observation target part. The acoustic lens 76 is formed of, for example, silicon resin (millable silicone rubber (HTV rubber), liquid silicone rubber (RTV rubber), and the like), butadiene resin, and polyurethane resin, and powders of titanium oxide, alumina, silica, and the like are mixed as necessary.

The FPC 60 is electrically connected to the electrodes of each ultrasound transducer 48. Each of the plurality of coaxial cables 56 is wired to the FPC 60 at one end thereof. Then, in a case where the ultrasound endoscope 12 is connected to the ultrasound processor device 14 through the ultrasound connector 32a, each of the plurality of coaxial cables 56 is electrically connected to the ultrasound processor device 14 at the other end (side opposite to the FPC 60 side).

In the present embodiment, the ultrasound endoscope 12 may comprise an endoscope side memory 58 (refer to Fig. 4). The endoscope side memory 58 stores driving times of the plurality of ultrasound transducers 48 in a case of ultrasound diagnosis. Strictly speaking, in the endoscope side memory 58, the cumulative driving time of the driving target transducer among the plurality of ultrasound transducers 48 is stored. The endoscope side memory 58 may further store a cumulative process time of the polarization process of the polarization target transducer among the plurality of ultrasound transducers 48.

In the present embodiment, an execution period of ultrasound diagnosis, that is, a period from the start of acquisition of an ultrasound image (moving image) to the end thereof (more specifically, a time during which ultrasound diagnosis is performed in the live mode), is set as the cumulative driving time and the cumulative process time. However, the present invention is not limited thereto, and the time for which the driving voltage is supplied to the driving target transducer may be set as the cumulative driving time, and the time for which the polarization voltage is supplied to the polarization target transducer may be set as the cumulative process time.

In a state in which the ultrasound endoscope 12 is connected to the ultrasound processor device 14, the CPU 152 of the ultrasound processor device 14 can access the endoscope side memory 58 to read the cumulative driving time and further the cumulative process time stored in the endoscope side memory 58. In addition, the CPU 152 of the ultrasound processor device 14 rewrites the cumulative driving time and further the cumulative process time stored in the endoscope side memory 58 to a default value, or updates the stored cumulative driving time and further the cumulative process time to a new cumulative driving time in a case where the cumulative driving time and the further cumulative process time change with the execution of ultrasound diagnosis.

### (Endoscope Observation Portion)

The endoscope observation portion 38 is a portion that is provided to acquire an endoscope image, and is arranged on a proximal end side than the ultrasound observation portion 36 in the distal end portion 40 of the insertion part 22. As shown in Figs. 2 and 3, the endoscope observation portion 38 includes an observation window 82, an objective lens 84, the solid-state imaging element 86, the illumination window 88, a cleaning nozzle 90, a wiring cable 92, and the like.

The observation window 82 is attached in a state obliquely inclined with respect to the axial direction (the longitudinal axis direction of the insertion part 22) in the distal end portion 40 of the insertion part 22. Light incident through the observation window 82 and reflected at the observation target adjacent part is formed on the imaging surface of the solid-state imaging element 86 by the objective lens 84.

The solid-state imaging element 86 photoelectrically converts reflected light of the observation target adjacent part transmitted through the observation window 82 and the objective lens 84 and formed on the imaging surface, and outputs an imaging signal. As the solid-state imaging element 86, it is possible to use a charge coupled device (CCD), a complementary metal oxide semiconductor (CMOS), and the like. The captured image signal output from the solid-state imaging element 86 is transmitted to the endoscope processor device 16 by the universal cord 26 through the wiring cable 92 extending from the insertion part 22 to the operating part 24.

The illumination windows 88 are provided at both side positions of the observation window 82. An exit end of a light guide (not shown) is connected to the illumination window 88. The light guide extends from the insertion part 22 to the operating part 24, and its incidence end is connected to the light source device 18 connected through the universal cord 26. The illumination light emitted from the light source device 18 is transmitted through the light guide and is emitted from the illumination window 88 toward the observation target adjacent part.

The cleaning nozzle 90 is an ejection hole formed in the distal end portion 40 of the insertion part 22 in order to clean the surfaces of the observation window 82 and the illumination windows 88, and air or a cleaning liquid is ejected from the cleaning nozzle 90 toward the observation window 82 and the illumination windows 88. In the present embodiment, the cleaning liquid ejected from the cleaning nozzle 90 is water, in particular, degassed water. However, the cleaning liquid is not particularly limited, and other liquids, for example, normal water (water that is not degassed) may be used.

### (Water Supply Tank and Suction Pump)

The water supply tank 21a is a tank that stores degassed water, and is connected to the light source connector 32c by an air/water supply tube 34a. Degassed water is used as a cleaning liquid ejected from the cleaning nozzle 90.

The suction pump 21b sucks aspirates (including degassed water supplied for cleaning) into the body cavity through the treatment tool outlet port 44. The suction pump 21b is connected to the light source connector 32c by a suction tube 34b. The ultrasound endoscope system 10 may comprise an air supply pump that supplies air to a predetermined air supply destination, or the like.

Inside the insertion part 22 and the operating part 24, the treatment tool channel 45 and an air/water supply pipe line (not shown) are provided.

The treatment tool channel 45 communicates a treatment tool insertion port 30 and the treatment tool outlet port 44 provided in the operating part 24. Furthermore, the treatment tool channel 45 is connected to a suction button 28b provided in the operating part 24. The suction button 28b is connected to the suction pump 21b in addition to the treatment tool channel 45.

The air/water supply pipe line communicates with the cleaning nozzle 90 on one end side, and is connected to an air/water supply button 28a provided in the operating part 24 on the other end side. The air/water supply button 28a is connected to the water supply tank 21a in addition to the air/water supply pipe line.

### (Operating part)

The operating part 24 is a portion that is operated by the operator in a case of a start of ultrasound diagnosis, during diagnosis, in a case of an end of diagnosis, and the like, and has one end to which one end of the universal cord 26 is connected. As shown in Fig. 1, the operating part 24 has the air/water supply button 28a, the suction button 28b, a pair of angle knobs 29, and a treatment tool insertion port (forceps port) 30.

In a case where each of a pair of angle knobs 29 is moved rotationally, the bendable portion 42 is remotely operated to be bent and deformed. By this deformation operation, the distal end portion 40 of the insertion part 22 in which the ultrasound observation portion 36 and the endoscope observation portion 38 are provided can be directed in a desired direction.

The treatment tool insertion port 30 is a hole formed in order that the treatment tool (not shown), such as forceps, is inserted thereinto, and communicates with the treatment tool outlet port 44 through the treatment tool channel 45. The treatment tool inserted into the treatment tool insertion port 30 is introduced into the body cavity from the treatment tool outlet port 44 after passing through the treatment tool channel 45.

The air/water supply button 28a and the suction button 28b are two-stage switching type push buttons, and are operated to switch opening and closing of the pipe line provided inside each of the insertion part 22 and the operating part 24.

### «Configuration of Ultrasound Processor Device»

The ultrasound processor device 14 causes m ultrasound transducers 48 (specifically, the driving target elements) among N ultrasound transducers 48 of the ultrasound transducer unit 46 to transmit and receive ultrasonic waves, and generates an ultrasound image by imaging the reception signal, which is output from the same m ultrasound transducers 48 (that is, the driving target elements) in a case of ultrasonic wave reception. In addition, the ultrasound processor device 14 displays the generated ultrasound image on the monitor 20.

Further, in the present embodiment, the ultrasound processor device 14, simultaneously, supplies the polarization voltage to, for example, (N - m) polarization target transducers among N ultrasound transducers 48 that are not used for generating the ultrasound image after being used for generating the ultrasound image, to polarize the polarization target transducers. In this manner, by performing the polarization process on the polarization target transducer that is not used for generating the ultrasound image, the depolarized ultrasound transducer 48 can be polarized again by repeating the ultrasound diagnosis. As a result, it is possible to restore the reception sensitivity of the ultrasound transducer 48 with respect to ultrasonic waves to a satisfactory level.

As shown in Fig. 4, the ultrasound processor device 14 has the multiplexer 140, the reception circuit 142, the transmission circuit 144, an A/D converter 146, an application specific integrated circuit (ASIC) 148, the cine memory 150, the central processing unit (CPU) 152, and a digital scan converter (DSC) 154.

The reception circuit 142 and the transmission circuit 144 are electrically connected to the ultrasound transducer array 50 of the ultrasound endoscope 12.

The multiplexer 140 selects a maximum of m driving target transducers from the N ultrasound transducers 48 and opens the channels thereof to generate the ultrasound image, and simultaneously selects, for the polarization process, a maximum of (N - m) driving target transducers, which are not the driving target transducers, from the N ultrasound transducers 48 and opens the channels thereof for polarization.

The transmission circuit 144 has a field programmable gate array (FPGA), a pulser (pulse generating circuit 158), a switch (SW), and the like, and is connected to the multiplexer 140 (MUX). Instead of the FPGA, an application specific integrated circuit (ASIC) may be used.

The transmission circuit 144 is a circuit that supplies the driving voltage for transmitting the ultrasonic wave to the driving target transducer selected by the multiplexer 140 in accordance with a control signal sent from the CPU 152 to transmit the ultrasonic wave from the ultrasound transducer unit 46 and that simultaneously supplies the polarization voltage for performing the polarization process on the polarization target transducer selected by the multiplexer 140 in accordance with the control signal sent from the CPU 152 to perform the polarization process on the polarization target transducer that is not selected as the driving target transducer among the plurality of ultrasound transducers 48 of the ultrasound transducer unit 46. The driving voltage is a pulsed voltage signal (transmission signal), and is applied to the electrodes of the driving target transducers through the universal cord 26 and the coaxial cable 56. The polarization voltage is also a pulsed voltage signal (transmission signal), and is applied to the electrodes of the driving target transducers through the universal cord 26 and the coaxial cable 56.

The transmission circuit 144 has the pulse generating circuit 158 that generates a transmission signal based on the control signal. Under the control of the CPU 152, the transmission circuit 144 generates a transmission signal for driving a plurality of ultrasound transducers 48 to generate ultrasonic waves using the pulse generating circuit 158 and supplies the transmission signal to a plurality of ultrasound transducers 48 to be driven, and generates a transmission signal for the polarization process and supplies the transmission signal to a plurality of ultrasound transducers 48 to be polarized.

That is, the transmission circuit 144 generates, under the control of the CPU 152, a first transmission signal having the driving voltage for performing the ultrasound diagnosis using the pulse generating circuit 158 to perform the ultrasound diagnosis, and simultaneously, under the control of the CPU 152, generates a second transmission signal having the polarization voltage for performing the polarization process using the same pulse generating circuit 158 as the case of generating the first transmission signal, to perform the polarization process. Here, it is preferable that a signal waveform of the first transmission signal for generating the ultrasonic wave for performing the ultrasound diagnosis is different from a signal waveform of the second transmission signal for the polarization process for performing the polarization process. The fact that the signal waveforms are different from each other means that at least one of a frequency, voltage, or wave number of the signal waveform is different.

The reception circuit 142 is a circuit that receives electric signals output from the driving target transducers, which receive the ultrasonic waves (echoes), that is, reception signals. In addition, according to the control signal sent from the CPU 152, the reception circuit 142 amplifies the reception signal received from the ultrasound transducer 48 and transmits the amplified signal to the A/D converter 146. The A/D converter 146 is connected to the reception circuit 142, and converts the reception signal received from the reception circuit 142 from an analog signal to a digital signal and outputs the converted digital signal to the ASIC 148.

The ASIC 148 is connected to the A/D converter 146. As shown in Fig. 4, the ASIC 148 is constituted of a phase matching unit 160, a B mode image generation unit 162, a PW mode image generation unit 164, a CF mode image generation unit 166, and a memory controller 151.

In the present embodiment, although the above-described functions (specifically, the phase matching unit 160, the B mode image generation unit 162, the PW mode image generation unit 164, the CF mode image generation unit 166, and the memory controller 151) are realized by a hardware circuit, such as the ASIC 148, the present invention is not limited thereto. The above-described functions may be realized by making the central processing unit (CPU) and software (computer program) for executing various kinds of data processing cooperate with each other.

The phase matching unit 160 executes processing of giving a delay time to the reception signals (reception data) digitized by the A/D converter 146 and performing phasing addition (performing addition after matching the phases of the reception data). By the phasing addition processing, a sound ray signal with narrowed focus of the ultrasound echo is generated.

The B mode image generation unit 162, the PW mode image generation unit 164, and the CF mode image generation unit 166 generate an ultrasound image based on the electric signal (strictly speaking, an audio signal generated by phasing and adding the reception data) that is output from the driving target transducer among the plurality of ultrasound transducers 48 in a case where the ultrasound transducer unit 46 receives the ultrasonic wave.

The B mode image generation unit 162 is an image generation unit that generates a B mode image as a tomographic image of the inside (the inside of the body cavity) of the patient. For the sequentially generated sound ray signals, the B mode image generation unit 162 corrects the attenuation due to the propagation distance according to the depth of the reflection position of the ultrasonic wave by sensitivity time gain control (STC). The B mode image generation unit 162 performs envelope detection processing and logarithm (Log) compression processing on the corrected sound ray signal, thereby generating a B mode image (image signal).

The PW mode image generation unit 164 is an image generation unit that generates an image indicating a speed of a blood flow in a predetermined direction. The PW mode image generation unit 164 extracts a frequency component by performing fast Fourier transform on a plurality of sound ray signals in the same direction among the sound ray signals sequentially generated by the phase matching unit 160. Thereafter, the PW mode image generation unit 164 calculates the speed of blood flow from the extracted frequency component, and generates a PW mode image (image signal) indicating the calculated speed of blood flow.

The CF mode image generation unit 166 is an image generation unit that generates an image indicating information regarding a blood flow in the predetermined direction. The CF mode image generation unit 166 generates an image signal indicating information regarding the blood flow by obtaining autocorrelation of a plurality of sound ray signals in the same direction among the sound ray signals sequentially generated by the phase matching unit 160. Thereafter, based on the image signal described above, the CF mode image generation unit 166 generates a CF mode image (image signal) as a color image in which the blood flow information is superimposed on the B mode image signal generated by the B mode image generation unit 162.

The memory controller 151 stores the image signal generated by the B mode image generation unit 162, the PW mode image generation unit 164, or the CF mode image generation unit 166 in the cine memory 150.

The DSC 154 is connected to the ASIC 148, converts (raster conversion) the signal of the image generated by the B mode image generation unit 162, the PW mode image generation unit 164, or the CF mode image generation unit 166 into an image signal compliant with a normal television signal scanning system, executes various kinds of necessary image processing, such as gradation processing, on the image signal, and then, outputs the image signal to the monitor 20.

The cine memory 150 has a capacity for accumulating an image signal for one frame or image signals for several frames. An image signal generated by the ASIC 148 is output to the DSC 154, and is stored in the cine memory 150 by the memory controller 151. In a freeze mode, the memory controller 151 reads out the image signal stored in the cine memory 150 and outputs the image signal to the DSC 154. As a result, an ultrasound image (still image) based on the image signal read from the cine memory 150 is displayed on the monitor 20.

The CPU 152 functions as a control unit (control circuit) that controls each unit of the ultrasound processor device 14, is connected to the reception circuit 142, the transmission circuit 144, the A/D converter 146, and the ASIC 148, and controls such circuits. Specifically, the CPU 152 is connected to the console 100, and controls each unit of the ultrasound processor device 14 according to examination information, control parameters, and the like input through the console 100.

The CPU 152 automatically recognizes the ultrasound endoscope 12 based on a method, such as Plug and Play (PnP), in a case where the ultrasound endoscope 12 is connected to the ultrasound processor device 14 through the ultrasound connector 32a. Thereafter, the CPU 152 also accesses the endoscope side memory 58 of the ultrasound endoscope 12 to read the cumulative driving time and further the cumulative process time stored in the endoscope side memory 58.

In addition, the CPU 152 accesses the endoscope side memory 58 in a case of the end of the ultrasound diagnosis, and updates the cumulative driving time and further the cumulative process time stored in the endoscope side memory 58 to a value obtained by adding the time required for the ultrasound diagnosis performed immediately before to the cumulative driving time and further the cumulative process time stored in the endoscope side memory 58.

In the present embodiment, the cumulative driving time and the cumulative process time are stored on the ultrasound endoscope 12. However, the present invention is not limited thereto, and the cumulative driving time and the cumulative process time may be stored on the ultrasound processor device 14 side for each ultrasound endoscope 12.

Meanwhile, the CPU 152 may control the multiplexer 140 to select all the ultrasound transducers 48 other than the driving target transducer as the polarization target transducer, but may access to the endoscope side memory 58 before the start of the ultrasound diagnosis to read the cumulative driving time of the ultrasound transducers 48 other than the driving target transducer stored in the endoscope side memory 58, and may control the multiplexer 140 to select the polarization target transducer according to the cumulative driving time read from the ultrasound transducers 48 other than the driving target transducer according to the read cumulative driving time.

In the present invention, the polarization driving pulse, which is the second transmission signal, is generated by the transmission circuit 144 that generates a diagnostic driving pulse, which is the first transmission signal for acquiring an ultrasound image. That is, the transmission circuit 144 has the same circuit configuration as an existing transmission circuit that does not have a new circuit configuration for generating the polarization driving pulse. Here, the transmission circuit 144 has a settable voltage range in which at least two driving voltages of an image driving voltage for a diagnostic driving pulse or a polarization driving voltage for a polarization driving pulse can be set. The driving voltage is set to the image driving voltage within the settable voltage range in the case of acquiring an ultrasound image, and the driving voltage is set to the polarization driving voltage, which is different from the image driving voltage, within the same settable voltage range in the case of performing polarization process. In the present invention, the polarization driving voltage is preferably set to voltage higher than the image driving voltage, more preferably to a higher voltage within the settable voltage range, and most preferably to the upper limit voltage.

Although the details will be described later, it is preferable that the polarization driving pulse (main lobe) is a driving pulse in a frequency band different from the probe frequency band of the diagnostic driving pulse.

Therefore, the driving voltage applied to the ultrasound transducer 48 in a case of polarization process is different from the driving voltage applied to the ultrasound transducer 48 in a case of acquisition of an ultrasound image, and it can be said that it is preferable that the driving voltage applied to the ultrasound transducer 48 in a case of polarization process is a higher voltage than the driving voltage applied to the ultrasound transducer 48 in a case of acquisition of an ultrasound image. In addition, the polarization driving pulse wave applied to the ultrasound transducer 48 in a case of polarization process is generated by the same transmission circuit 144 as for the diagnostic driving pulse wave applied to the ultrasound transducer 48 in a case of acquisition of an ultrasound image, and it can be said that it is preferable that the polarization driving pulse wave has a polarization driving voltage different from that of the image driving pulse wave within the same settable voltage range as the image driving pulse wave, and is a driving pulse having a frequency different from the probe frequency band for acquiring an ultrasound image.

From the above, the present invention has an existing transmission circuit configuration. And it can be said that in the present invention, using the transmission circuit 144 for the same driving pulse output as acquisition of an ultrasound image, a polarization driving pulse, which has a driving voltage within the same settable voltage range as the diagnostic driving pulse for acquiring an ultrasound image and has a frequency different from the probe frequency band, and a diagnostic driving pulse are simultaneously output, and polarization process of the ultrasound transducer 48 of the ultrasound endoscope 12 is performed simultaneously with a time for acquiring an ultrasound image.

The magnitude (voltage value or potential) of the driving voltage of the polarization driving pulse is set to an appropriate value satisfying the conditions for obtaining the repolarization effect, within the settable voltage range of the transmission circuit 144, by the CPU 152 in accordance with the specification of the ultrasound transducer 48 (specifically, the thickness and the material of the ultrasound transducer 48) provided in the ultrasound endoscope 12 connected to the ultrasound processor device 14. The supply time of the driving voltage of the polarization driving pulse is set to an appropriate value satisfying the conditions for obtaining the repolarization effect by the CPU 152 in accordance with the specification of the ultrasound transducer 48 (specifically, the thickness and the material of the ultrasound transducer 48). Thereafter, the CPU 152 performs polarization process based on the set values described above.

That is, in the present invention, the CPU (control circuit) 152 controls the transmission circuit 144 (pulse generating circuit 158) to generate diagnostic driving pulses (first transmission signal) to be applied to each of a plurality of first ultrasound transducers 48 that generate ultrasonic waves for acquisition of an ultrasound image.

Simultaneously, in order to perform polarization process of a plurality of second ultrasound transducers 48 that do not generate the ultrasonic waves, the CPU (control circuit) 152 controls a transmission circuit 144 to generate a polarization driving pulse (second transmission signal), which has a frequency different from the probe frequency band as an ultrasound probe (ultrasound transducer unit 46) for acquiring an ultrasound image and has a polarization driving voltage different from that of the diagnostic driving pulse within the same settable voltage range as the diagnostic driving pulse.

As a result, in the present invention, the diagnostic driving pulse is applied to the plurality of first ultrasound transducers 48, ultrasonic waves are generated from the plurality of first ultrasound transducers 48 by the diagnostic driving pulse, the polarization driving pulse is applied to the plurality of second ultrasound transducers 48, and the polarization process of the plurality of second ultrasound transducers 48 is performed by the polarization driving pulse.

Next, a pulse waveform and a driving waveform (transmission waveform) of a polarization driving pulse (transmission wave for polarization) transmitted from the transmission circuit 144 to the ultrasound transducer 48 in the present invention will be described.

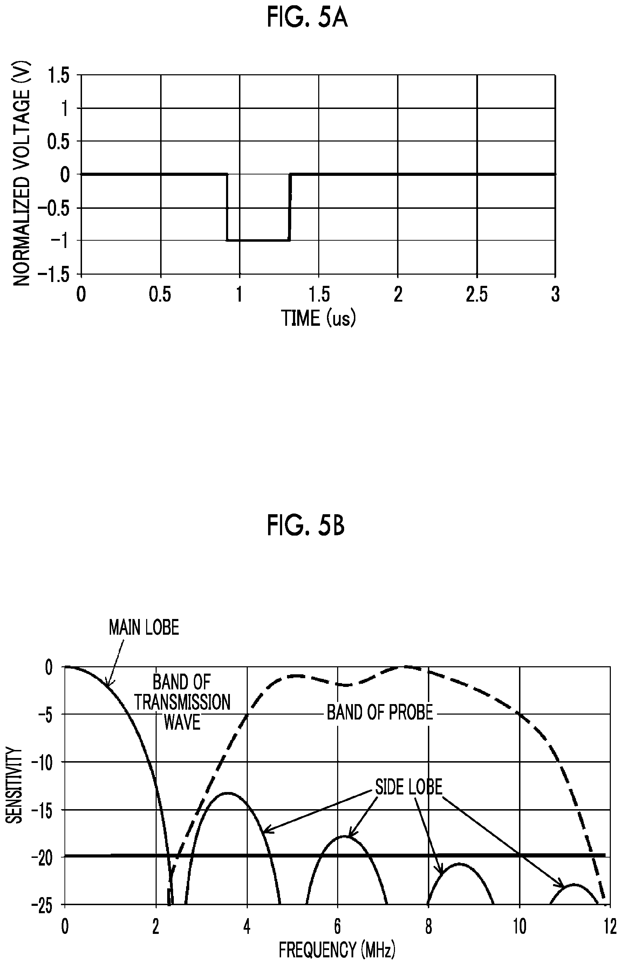

Figs. 5A and 5B are graphs of an example of a driving waveform of a polarization driving pulse transmitted from the transmission circuit shown in Fig. 4, and are graphs showing the relationship between the sensitivity and the frequency of the driving waveform. The driving waveform shown in Fig. 5A is a waveform of one unipolar wave having a frequency of 1.25 MHz.

In the present invention, the driving waveform of the polarization driving pulse is not particularly limited but has a unipolar waveform shown in Fig. 5A, and it is preferable to perform polarization process of the ultrasound transducer 48 using a polarization driving pulse having a driving waveform having a frequency characteristic shown by the solid line in Fig. 5B. In the example shown in Fig. 5B, for example, at a sensitivity level of -20 dB or more, the probe frequency band for acquiring an ultrasound image is about 2.7 MHz to about 11.7 MHz as shown by the broken line, while the band of the main lobe of the driving waveform of the polarization driving pulse shown by the solid line is about 2.3 MHz or less. That is, the band characteristic of the frequency of the polarization driving pulse and the band characteristic of the frequency of the diagnostic driving pulse do not overlap each other at a sensitivity level of -20 dB or more.

That is, in the present invention, as shown in Fig. 5B, in the driving waveform of the polarization driving pulse, it is preferable that the frequency band of the main lobe and the probe frequency band shown by the broken line do not overlap each other at a sensitivity level of -20 dB or more. In addition, it is preferable that the frequency band of the main lobe is lower than the probe frequency band at a sensitivity level of -20 dB or more. The reason is that, in the polarization process, it is necessary to reduce an influence on the ultrasound image by preventing excessive ultrasound output and to reduce an influence on the body cavity of the subject due to temperature rise by preventing the temperature rise. In particular, the upper limit temperature of the distal end portion of the ultrasound endoscope 12 inserted into the body cavity of the subject is strictly limited so as not to affect the body cavity and the like, and it is necessary to prevent the temperature rise.

In the present invention, since the polarization driving pulse (main lobe) is transmitted outside the probe frequency band, the energy input to the ultrasound transducer 48 is reduced. Therefore, the temperature rise can be suppressed. In addition, since the outside of the probe frequency band is the outside of a resonance band in which the ultrasound transducer 48 resonates. Accordingly, the output sound pressure is reduced even though the polarization driving pulse (main lobe) is applied to the ultrasound transducer 48.

In the driving waveform of the polarization driving pulse shown in Fig. 5B, it can be seen that, in addition to the main lobe, within the probe frequency band, accompanied by the main lobe, one or more side lobes similarly shown by the solid line (in the example shown in Fig. 5B, four side lobes) are generated. As shown in Fig. 5B, it is preferable that all the maximum sensitivities of the side lobes within the probe frequency band are equal to or less than -10 dB and the average sensitivity of the side lobes is equal to or less than -20 dB. The reason is as follows.

In general, the specification of the frequency characteristic of the probe is expressed in the -20 dB band of the transmission and reception sensitivity. This is because it is determined that the signal of 1/10 or less from the peak of the sensitivity hardly affects an image. On the other hand, the band of the transmission wave is different from that in the case of the probe. Since only a transmission portion is taken into consideration, the level of 20 dB/2 = 10 dB is the threshold value. For this reason, -10 dB is more preferable in a case where a transmission component is considered.

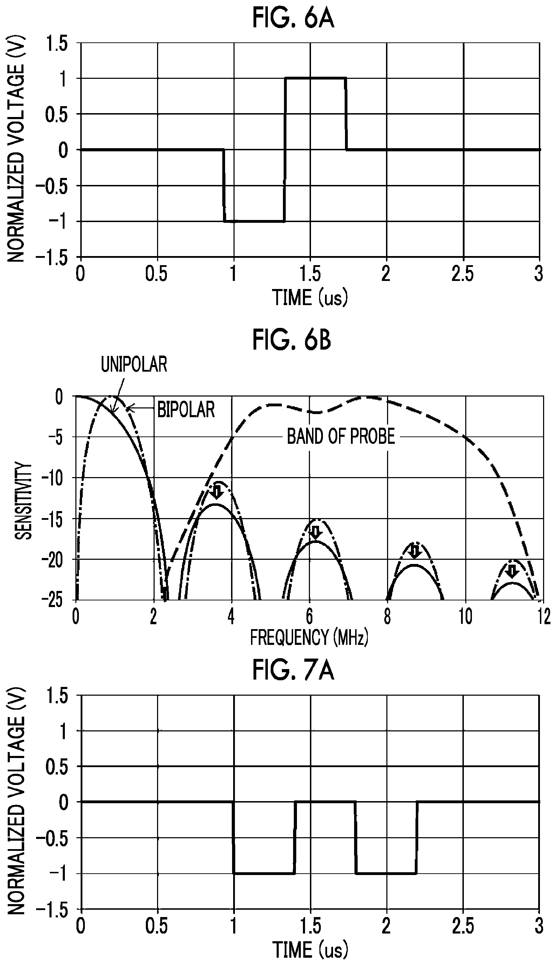

In the present invention, the driving waveform of the polarization driving pulse is not particularly limited, and may be a bipolar waveform shown in Fig. 6A. However, the driving waveform of the polarization driving pulse is preferably a unipolar waveform as shown in Fig. 5A. The reason is that, as in the frequency characteristic of the driving waveform shown in Fig. 6B, the sensitivity of the main lobe does not change whether the driving waveform is a unipolar waveform shown by the solid line or a bipolar waveform shown by the one-dot chain line, but the sensitivities of all of the four side lobes in the case of the unipolar waveform are lower than those in the case of the bipolar waveform.

Therefore, by forming the transmission waveform as a unipolar waveform as shown in Fig. 5A, not only the main lobe but harmonic components can be suppressed. As a result, higher effects can be expected.

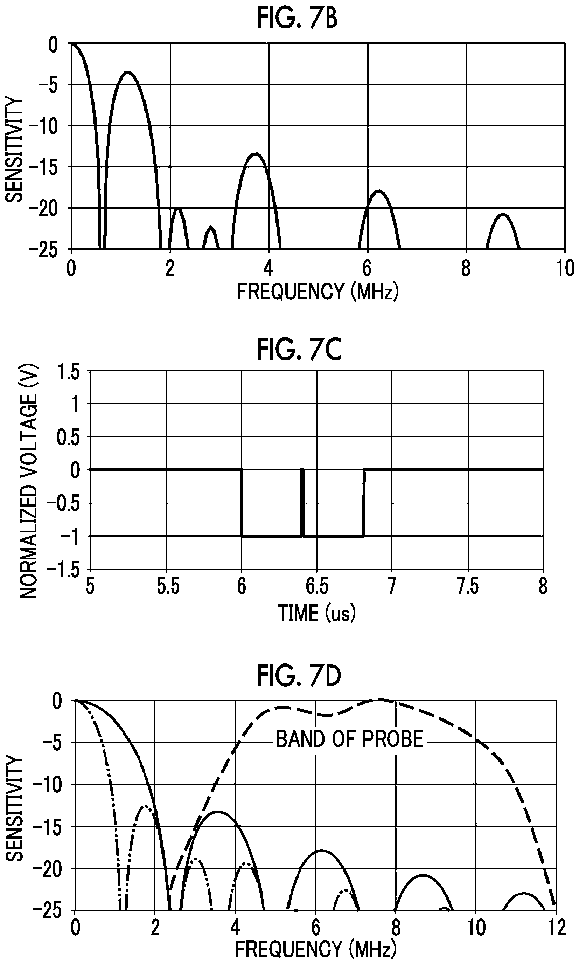

As shown in Fig. 7A, a plurality of unipolar waveforms may be transmitted as the polarization driving pulses. In the example shown in Fig. 7A, two pulse waves may be transmitted. The polarization driving pulse shown in Fig. 7A has a driving waveform including two pulse waves as a driving waveform of the polarization driving pulse shown in Fig. 5A. The frequency characteristic of the driving waveform of the polarization driving pulse shown in Fig. 7A is shown in Fig. 7B. The frequency characteristic of the driving waveform shown in Fig. 7B is different from the frequency characteristic of the driving waveform shown in Fig. 5B in terms of the waveform of the main lobe, but the waveform of the side lobe does not change much.

In addition, as shown in Fig. 7C, it is preferable to transmit a polarization driving pulse in which a plurality of pulse waveforms are connected to each other with the time of the minimum number of clocks between driving waveforms of the polarization driving pulse as unipolar waveforms. That is, in the present invention, it is preferable that the transmission circuit 144 outputs a plurality of unipolar waveforms as polarization driving pulses with the time of the minimum number of clocks defined in the ultrasound processor device 14 as an interval between the unipolar waveforms.

The reason is that it is optimal to apply a DC voltage for polarization process, but the DC voltage cannot be transmitted in a case where the transmission circuit 144 having an existing transmission circuit configuration is used as in the present invention.

The minimum and maximum time widths are determined depending on the type of pulser (pulse generating circuit 158) of the transmission circuit 144 of the ultrasound processor device 14 used in the ultrasound endoscope system 10. Therefore, by using the time of the minimum number of clocks defined in the transmission circuit 144 as the minimum time width, a high repolarization effect can be expected by putting the minimum time width between a plurality of unipolar waveforms so that a polarization process waveform close to a DC voltage is obtained. The minimum time width between two unipolar pulse waveforms, that is, the strongest pulse width is determined by the specification of the pulser (pulse generating circuit 158) of the transmission circuit 144. Control to comply with this specification is provided from the above-described FPGA in the transmission circuit 144.

As shown by a two-dot chain line in Fig. 7D, by using a combination of a plurality of unipolar waveforms shown in Fig. 7C as the driving waveform of the polarization driving pulse, it is possible to reduce the maximum sensitivity of the side lobe more than in the case of the driving waveform of the polarization driving pulse including one unipolar waveform shown by the solid line in Fig. 7D.

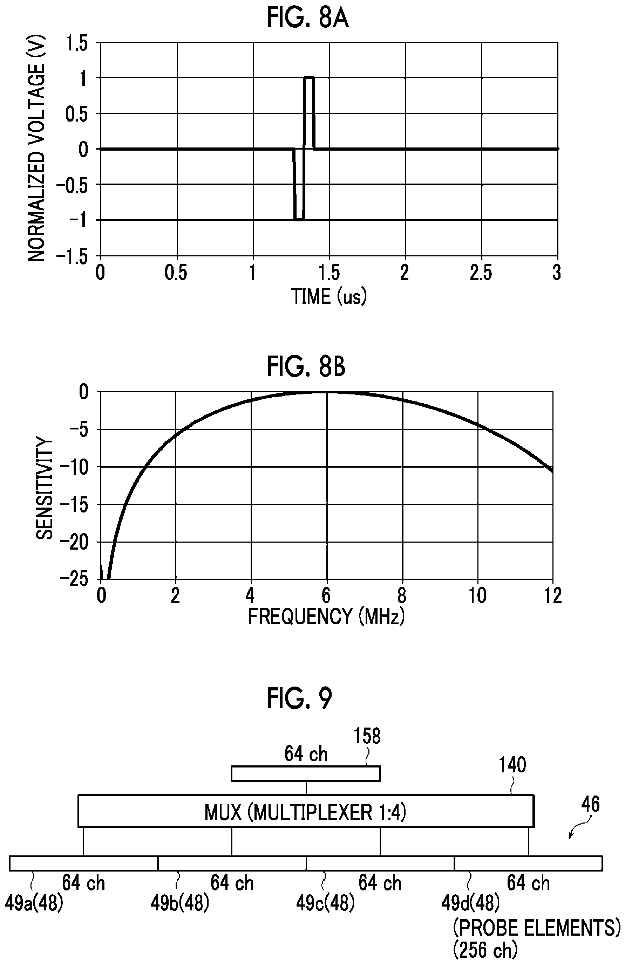

On the other hand, Figs. 8A and 8B are graphs of an example of a driving waveform of a diagnostic driving pulse transmitted from the transmission circuit shown in Fig. 4, and are graphs showing the relationship between the sensitivity and the frequency of the driving waveform. The driving waveform shown in Fig. 8A is a waveform of one bipolar wave having a center frequency of 6 MHz. The frequency characteristic of the driving waveform of the diagnostic driving pulse is shown in Fig. 8B.

From the above, it is preferable that the driving waveform (transmission waveform) of the polarization driving pulse is different from the driving waveform (transmission waveform) of the diagnostic driving pulse, and specifically, it is preferable that at least one of the voltage, the frequency, or the wave number is different.

Meanwhile, an ultrasound system, particularly a small-sized ultrasound system is reduced in size by limiting the transmission simultaneous opening number (the number of channels of pulser), that is, the ultrasound transducer that simultaneously transmits the ultrasonic wave. In a case of driving an ultrasound transducer array having multiple elements (a large number of ultrasound transducers), as shown in Fig. 9, the driving of multiple elements is realized through the multiplexer 140 that functions as a switcher. Meanwhile, in a high-end machine, the improvement of image quality is achieved by increasing the number of channels of the pulser.

That is, in the example shown in Fig. 9, while the number of channels of the elements (ultrasound transducers 48) of the probe (ultrasound transducer unit 46) is 256 channels (ch), that is, there are multiple elements, the number of channels of pulser (pulse generating circuit 158) on the system side is 64 ch. Therefore, an element group 49 consisting of the elements 48 of 256 ch of the probe 46 is divided into four element groups 49a to 49d consisting of the elements 48 of 64 ch, the one divided element group 49a consisting of the elements 48 of 64 ch is selected by the multiplexer 140, each element 48 of the element group 49a is connected to each of 64 ch of the pulser 158, and the elements 48 of 64 ch are simultaneously opened and driven to transmit, thereby acquiring the ultrasound image. In the example shown in Fig. 9 and the examples shown in the following drawings, each element (ultrasound transducer) 48 in the element group is not shown.

Therefore, in the example shown in Fig. 9, the elements 48 of 64 ch of each of the remaining three element groups 49b to 49d are not driven and are not used for acquiring the ultrasound image. Also, after driving each element 48 of the element group 49a for a predetermined time to acquire the ultrasound image, the driving of each element 48 of the element group 49a can be stopped, and the elements 48 of 64 ch of the next element group 49b can be driven. Similarly, subsequently, the respective elements 48 of the element groups 49c and 49d can be sequentially driven.

Meanwhile, even in this case, the three element groups among the four element groups 49a to 49d are not driven, and thus the example shown in Fig. 9 is over-specified. Of course, in a case where the number of channels of the pulser 158 on the system side is 256 ch, all the elements 48 can be driven, and thus the multiplexer 140 is not required. Therefore, in a case where the number of channels of the elements 48 of the probe 46 is large, it is conceivable to increase the number of channels of the pulser 158 on the system side.

Meanwhile, the ultrasound transducer 48 of the ultrasound endoscope 12 is often formed of a small-diameter convex, and in this case, even in a case where the number of channels of the pulser 158 on the system side is increased to increase the simultaneous opening number of the ultrasound transducer 48, the contribution to the improvement in image quality of the element (ultrasound transducers) 48 at the end portion is small, and rather the increase of the number of channels of the pulser 158 on the system side may be a factor causing artifacts such as side lobes. Therefore, the transmission is often performed by limiting the transmission opening number to a range of ±45 degrees.

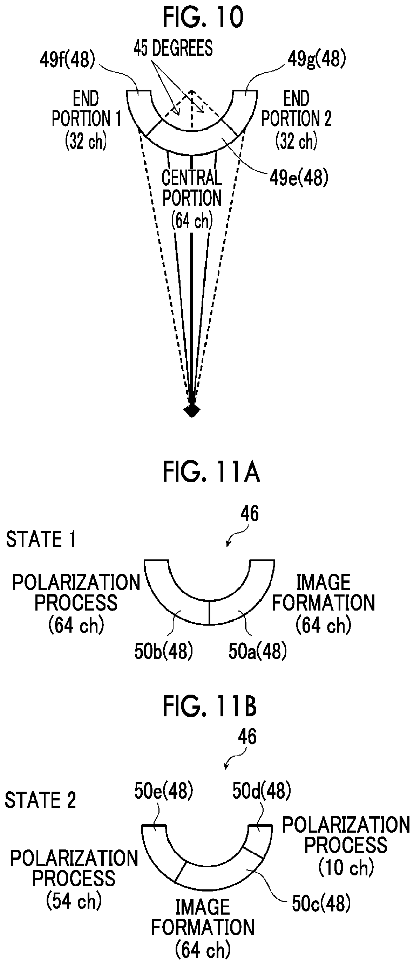

For example, in a microconvex transducer shown in Fig. 10, elements 48 of 64 ch of a central element group 49e are driven to form a thick line at a center, but elements 48 of 32 ch of each of element groups 49f and 49g at both end portions 1 and 2 are not driven.

In this way, the ultrasound endoscope system designed for an ultrasound endoscope is often designed with the minimum necessary number of elements (for example, 64 ch). However, in the ultrasound endoscope system that also uses the probe for body surface, the simultaneous opening number may be excessive.

Therefore, in the present invention, by simultaneously performing the polarization process with image visualization, that is, acquisition of the ultrasound image using the excessive pulser, the polarization process can be performed without a decrease of frame rate.

That is, by applying the present invention to the example shown in Fig. 10, and under the control of the CPU 152, while the elements 48 of 64 ch of the central element group 49e are driven to acquire an ultrasound image, simultaneously, the polarization process can be performed on the elements 48 of 32 ch of each of the element groups 49f and 49g at both end portions 1 and 2.

Although it is over-specified, by applying the present invention to the example shown in Fig. 9, and under the control of the CPU 152, while the elements 48 of 64 ch of the element group 49a are driven to acquire an ultrasound image, simultaneously, the polarization process can be performed on each element 48 of 64 ch of the three remaining element groups 49b to 49d.

That is, in the present invention, by performing a repolarization process (also simply referred to as polarization process) on the channel of the element 48 that simultaneously opens but is not used for acquiring the ultrasound image, the acquisition of the ultrasound image and the repolarization process are simultaneously performed.

Hereinafter, examples in which the present invention is applied will be described, but these examples are merely examples, and the present invention is not limited thereto.

### (Example 1)

Figs. 11A to 11C are diagrams showing a state where under control of the CPU (control circuit) 152, while the element (ultrasound transducer) 48 driven for acquisition of the ultrasound image, that is, the image formation is shifted, the image formation and the polarization process are simultaneously performed.

In all of the states shown in Figs. 11A to 11C, a state where the polarization process is performed on the element (ultrasound transducer) 48 that is not used for the image formation by using the pulser 158 that is not used for the image formation is shown. In the polarization process, it is preferable to use voltage different from the voltage applied to the element 48 used for the image formation.

First, in a state 1 shown in Fig. 11A, elements 48 of 64 ch of an element group (array) 50a of the right half of elements 48 of total of 128 ch of the probe 46 are used for transmitting the ultrasonic wave for the image formation, and elements 48 of 64 ch of an element group 50b of the left half are subjected to the polarization process.

Next, in a state 2 shown in Fig. 11B, on elements 48 of 10 ch on the right side in the elements 48 of 64 ch of the element group 50a of the right half shown in Fig. 11A, the transmission of the ultrasonic wave for the image formation is stopped and the polarization process is started, and on elements 48 of 10 ch on the right side in the elements 48 of 64 ch of the element group 50b of the left half shown in Fig. 11A, the polarization process is stopped and the transmission of the ultrasonic wave for the image formation is started. Therefore, in the state 2 shown in Fig. 11B, elements 48 of 64 ch of a central element group 50c of the elements 48 of total of 128 ch of the probe 46 are used for the image formation, but the elements 48 of 10 ch of an element group (array) 50d on the right side and elements 48 of 54 ch of an element group (array) 50e on the left side are subjected to the polarization process.

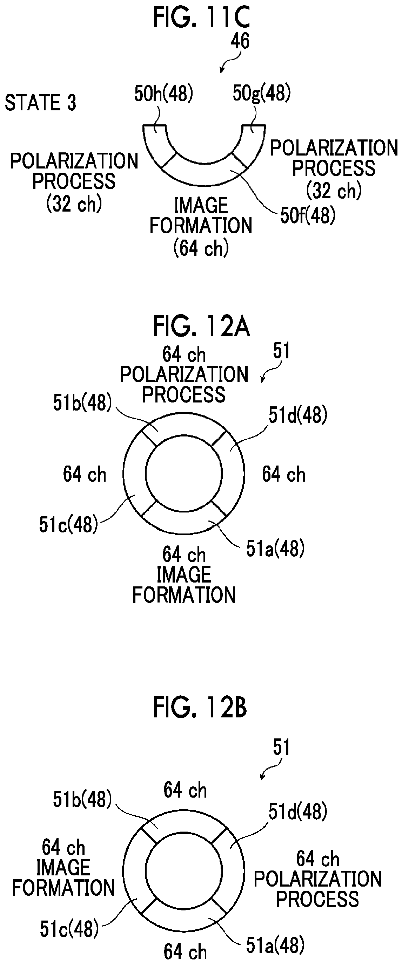

Next, in a state 3 shown in Fig. 11C, on elements 48 of 22 ch on the right side in the elements 48 of 64 ch of the central element group 50c shown in Fig. 11B, the transmission of the ultrasonic wave for the image formation is stopped and the polarization process is started, and on elements 48 of 22 ch on the right side in the elements 48 of 54 ch of the element group 50e of the left half shown in Fig. 11B, the polarization process is stopped and the transmission of the ultrasonic wave for the image formation is started. Therefore, in the state 3 shown in Fig. 11C, elements 48 of 64 ch of a central element group 50f of the elements 48 of total of 128 ch of the probe 46 are used for the image formation, but the elements 48 of 32 ch of an element group (array) 50g on the right side and elements 48 of 32 ch of an element group (array) 50h on the left side are subjected to the polarization process.

As in the states 1, 2, and 3 shown in Figs. 11A to 11C, it is possible to change the position of the element 48 on which the polarization process is performed in accordance with the shift of the element 48 used for transmitting and receiving ultrasonic wave for the image formation.

In the examples shown in Figs. 11A to 11C, the position of the element 48 on which the polarization process is performed is also changed in accordance with the shift of the element 48 used for transmitting and receiving ultrasonic wave for the image formation, but the number of channels of the elements 48 used for the image formation and the number of channels of the elements 48 on which the polarization process is performed are not changed, and are both 64 ch.

Here, in a case where the image formation and the polarization process are simultaneously performed, in the image formation, the transmission and reception of ultrasonic wave to and from the element 48 are required, but in the polarization process, only the transmission is required, and thus the reception of the reception signal is not required.

In a case where the simultaneous opening number of the elements 48 used for the image formation is changed in association with the change of a focus position for observation, in accordance with the change of the simultaneous opening number, it is preferable to change the number of channels (simultaneous opening number) of the elements 48 on which the polarization process is performed.

For example, in a case where, from a state where both the number of channels in the image formation and the number of channels in the polarization process are 64 ch, the number of simultaneous opening channels of the elements 48 used for the image formation is changed to 56 ch in accordance with the change of the focus position for observation, it is preferable to change the number of channels of the elements 48 on which the polarization process is performed to 72 ch.

In addition, as described above, in the image formation and the polarization process, it is preferable to use different voltages of the transmission signal (driving pulse) applied to the element 48, and also for transmission frequency, it is preferable to use different waveforms in the image formation and the polarization process. In addition, it is preferable that the wave number of the transmission waveform is also different.

For this reason, it is desirable that the transmission circuit 144 has two systems for voltage, and it is preferable that one system is used for the image formation and the other system is used for the polarization process to apply different voltages. Instead of this, it is also possible to attenuate either one in one system.

### (Example 2)

Figs. 12A and 12B are diagrams showing a state where in a case where a radial transducer array 51, which is an ultrasound transducer array in which the plurality of ultrasound transducers are arranged in a circumferential shape, is used, a center of the plurality of ultrasound transducers for the image formation and a center of the plurality of ultrasound transducers for the polarization process are arranged at positions different from each other by 180 degrees.

First, in a state shown in Fig. 12A, elements 48 of 64 ch of a lower element group (array) 51a of elements 48 of total of 256 ch of the probe 46 are used for transmitting and receiving the ultrasonic wave for the image formation, and elements 48 of 64 ch of an upper element group 51b are subjected to the polarization process. As shown in Fig. 12A, the lower element group 51a and the upper element group 51b are arranged at point-symmetric positions with respect to a center of the radial transducer array 51, and a center of the elements 48 of the lower element group 51a and a center of the elements 48 of the upper element group 51b are arranged at positions different from each other by 180 degrees. In Fig. 12A, a left element group 51c and a right element group 51d arranged at point-symmetric positions with respect to the center of the radial transducer array 51 are in a freeze state where the left element group 51c and the right element group 51d are neither used for transmitting and receiving the ultrasonic wave nor subjected to the polarization process.

Next, in a state shown in Fig. 12B, the radial transducer array 51 is shifted by 90 degrees clockwise from the state shown in Fig. 12A, the elements 48 of 64 ch of the left element group 51c of the elements 48 of total of 256 ch of the probe 46 are used for transmitting and receiving the ultrasonic wave for the image formation, and elements 48 of 64 ch of the right element group 51d arranged at a point-symmetric position with respect to the center of the radial transducer array 51 are subjected to the polarization process. That is, even in the state shown in Fig. 12B, the left element group 51c for transmitting and receiving the ultrasonic wave and the right element group 51d for the polarization process are arranged at point-symmetric positions with respect to a center of the radial transducer array 51, and a center of the elements 48 of the lower element group 51a and a center of the elements 48 of the upper element group 51b are arranged at positions different from each other by 180 degrees. In Fig. 12B, the lower element group 51a and the upper element group 51b arranged at point-symmetric positions with respect to the center of the radial transducer array 51 are in a freeze state.

As described above, it is preferable that the plurality of elements 48 of the radial transducer array 51 are controlled such that the centers of the simultaneous transmission openings on which the image formation and the polarization process are performed maintain 180 degrees from each other. Accordingly, in the plurality of elements 48 of the radial transducer array 51, a scan for the polarization process can be inserted in a direction different by 180 degrees with respect to a scan for the image formation.

In addition, as described above, the transmission for the polarization process is outside the band of the probe, and has almost no influence on the image formation.

Therefore, as shown in Figs. 11A to 11C, since the transmission for the polarization process has almost no influence on the image formation, the element 48 for the image formation and the element 48 for the polarization process may be adjacent to each other. Meanwhile, to further reduce the influence on the image formation such as crosstalk and noise, it is preferable that at least two to three elements 48 in a freeze state are interposed between the element 48 for the image formation and the element 48 for the polarization process.

Further, as described above, as shown in Figs. 12A and 12B, in the radial transducer array 51, by arranging the center of the plurality of elements 48 for the image formation and the center of the plurality of elements 48 for the polarization process at positions of 180 degrees, the influence on the image formation can be further reduced.

### <<Operation Example of Ultrasound Endoscope System>>

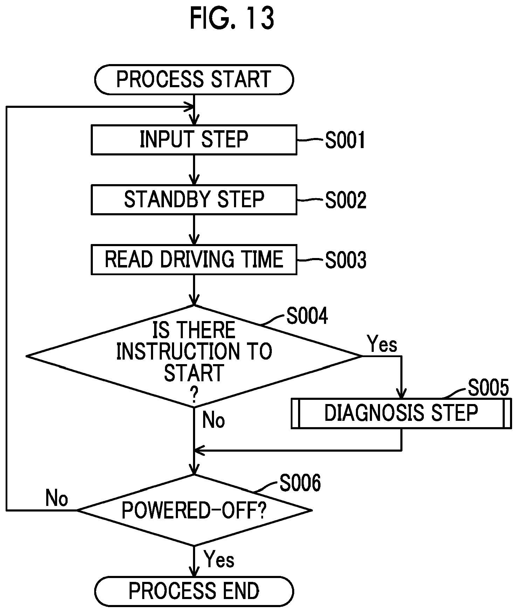

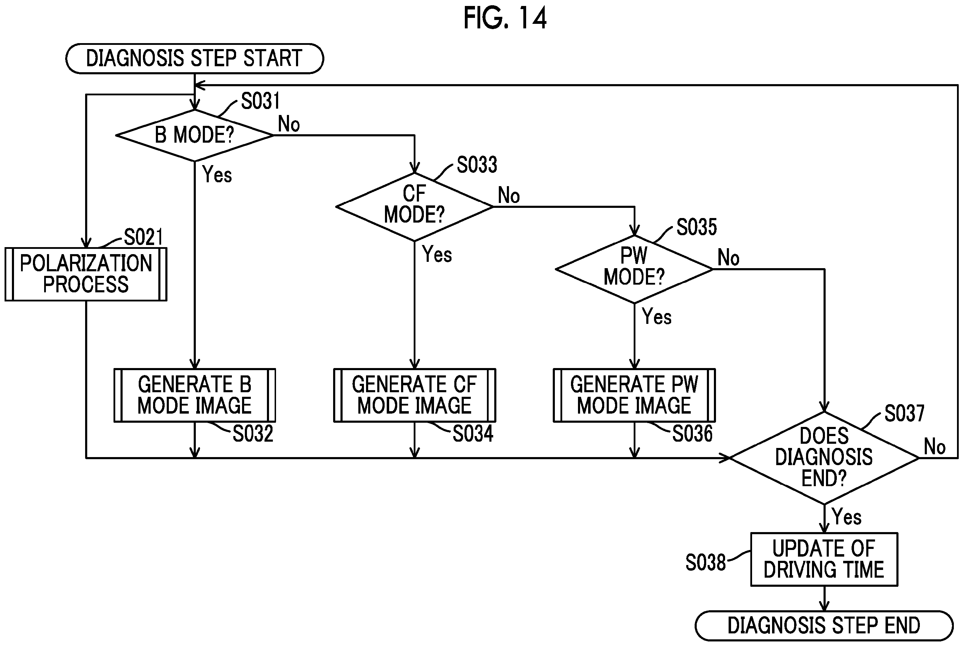

Next, as an operation example of the ultrasound endoscope system 10, a flow of a series of process (hereinafter, also referred to as diagnosis process) regarding ultrasound diagnosis will be described with reference to Figs. 13 and 14. Fig. 13 is a flowchart showing a flow of diagnosis process using the ultrasound endoscope system 10. Fig. 14 is a flowchart showing a procedure of a diagnosis step during the diagnosis process.

In a case where each unit of the ultrasound endoscope system 10 is powered on in a state in which the ultrasound endoscope 12 is connected to the ultrasound processor device 14, the endoscope processor device 16, and the light source device 18, the diagnosis process starts with the power-ON as a trigger. In the diagnosis process, as shown in Fig. 13, an input step is performed first (S001). In the input step, the operator inputs examination information, control parameters, and the like through the console 100. In a case where the input step is completed, a standby step is performed until there is an instruction to start diagnosis (S002). Using the standby step, the CPU 152 of the ultrasound processor device 14 reads a cumulative driving time and a cumulative process time from the endoscope side memory 58 of the ultrasound endoscope 12 (S003).

Then, in a case where there is an instruction to start diagnosis from the operator (Yes in S004), the CPU 152 controls each unit of the ultrasound processor device 14 to perform a diagnosis step (S005). The diagnosis step proceeds along the flow shown in Fig. 14. In a case where the designated image generation mode is the B mode (Yes in S031), each unit of the ultrasound processor device 14 is controlled so as to generate a B mode image (S032). In a case where the designated image generation mode is not the B mode (No in S031) but the CF mode (Yes in S033), each unit of the ultrasound processor device 14 is controlled so as to generate a CF mode image (S034). Further, in a case where the designated image generation mode is not the CF mode (No in S033) but the PW mode (Yes in S035), each unit of the ultrasound processor device 14 is controlled so as to generate a PW mode image (S036). In a case where the designated image generation mode is not the PW mode (No in S036), the process proceeds to step S037.

Meanwhile, the polarization process step is started with the start of the diagnosis step as a trigger, and each unit of the ultrasound processor device 14 is controlled such that the polarization process is performed until the diagnosis step ends (S021).

That is, the CPU 152 controls each unit of the ultrasound processor device 14 so that polarization process is performed on the plurality of ultrasound transducers 48, which are a polarization target transducer in which the transmission of the ultrasonic waves for performing the ultrasound diagnosis and the reception of the reflected waves are not performed, simultaneously with the execution period of the ultrasound diagnosis, that is, during the diagnosis step, that is, in each frame time in which the transmission of the ultrasonic waves for performing the ultrasound diagnosis and the reception of the reflected waves are performed on the plurality of ultrasound transducers 48, which are a driving target transducer, and an image of each frame of an ultrasound image is acquired. That is, the acquisition of the ultrasound image and the polarization process are simultaneously performed during the execution period of the ultrasound diagnosis.

Subsequently, returning to Fig. 14, the CPU 152 determines whether or not the ultrasound diagnosis ends (S037). In a case where the ultrasound diagnosis does not end (No in S037), the process returns to the diagnosis step S031, and the generation of an ultrasound image in each image generation mode is repeatedly performed until the diagnosis end conditions are satisfied. In this case, the polarization process is also simultaneously performed. As the diagnosis end conditions, for example, the operator gives an instruction to end the diagnosis through the console 100.

On the other hand, in a case where the diagnosis end conditions are satisfied and accordingly the ultrasound diagnosis ends (Yes in S037), the polarization process also simultaneously ends, the CPU 152 adds the time required for the ultrasound diagnosis performed so far to the cumulative driving time and the cumulative process time read out from the endoscope side memory 58 in step S003, and updates the cumulative driving time and the cumulative process time stored in the endoscope side memory 58 to the cumulative driving time and the cumulative process time after the addition (S038). The diagnosis step ends at a point in time at which the series of steps (S031 to S038) in the diagnosis step end.

Subsequently, returning to Fig. 13, in a case where the respective units of the ultrasound endoscope system 10 are powered-off (Yes in S006), the diagnosis process ends. On the other hand, in a case where the respective units of the ultrasound endoscope system 10 are maintained in a powered-on state (No in S006), the process returns to the input step S001, and the respective steps of the diagnosis process described above are repeated.

Meanwhile, the polarization process may be performed on all the ultrasound transducers 48 corresponding to the polarization target transducers on which the transmission of the ultrasonic waves for performing ultrasound diagnosis and the reception of the reflected waves are not performed. However, even in a case where the ultrasound transducer 48 in which the cumulative process time of the polarization process has reached a predetermined time corresponds to the polarization target transducer, the polarization process may not be performed. That is, the ultrasound transducer 48 in which the cumulative process time of the polarization process has reached a predetermined time may not be subjected to the polarization process from the beginning, or the polarization process may be stopped at a point in time at which the cumulative process time of the polarization process has reached the predetermined time.

Since the dipoles applied to both surfaces of the ultrasound transducer 48 are reduced and depolarization proceeds according to the time at which the transmission of the ultrasonic wave for performing the ultrasound diagnosis and the reception of the reflected wave are performed, that is, the cumulative driving time of the plurality of ultrasound transducers 48, even during the image formation, the ultrasound transducer 48 in which the cumulative driving time has reached a predetermined time or more may be excluded from the driving target transducer on which the transmission of the ultrasonic wave for performing the ultrasound diagnosis and the reception of the reflected wave are performed, and the polarization process may be performed on the excluded ultrasound transducer 48 as the polarization target transducer.

The ultrasound endoscope system 10 can acquire an ultrasound image and an endoscope image and display the ultrasound image and the endoscope image on the monitor 20 in various display modes.

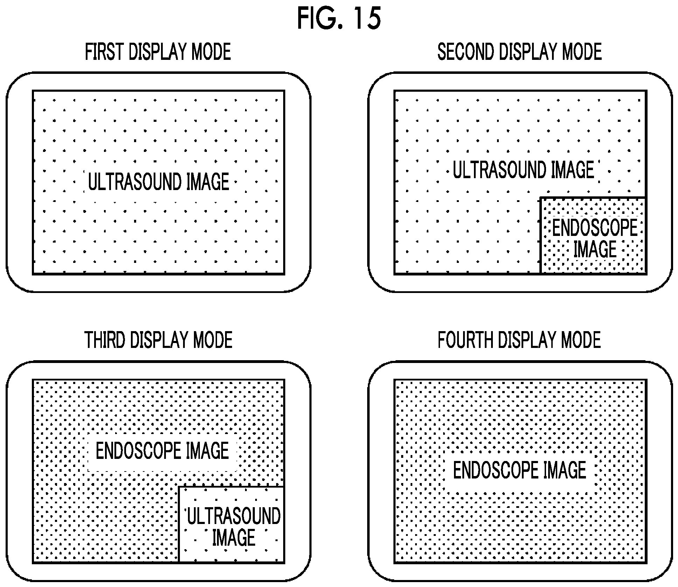

As shown in Fig. 15, the display modes include a first display mode in which only an ultrasound image is displayed, a second display mode in which an ultrasound image is displayed so as to be larger than an endoscope image by using picture in picture (PinP), a third display mode in which an ultrasound image is displayed so as to be smaller than an endoscope image by using the PinP similarly, and a fourth display mode in which only an endoscope image is displayed. The first to fourth display modes can be freely switched and displayed according to the user's instruction.

### <<Effectiveness of Ultrasound Endoscope System 10 according to Embodiment of Invention>>

While the ultrasound endoscope system 10 performs, during the execution period of the ultrasound diagnosis, the transmission of the ultrasonic wave for performing the ultrasound diagnosis and the reception of the reflected wave using the ultrasound transducer and acquires the image of each frame, simultaneously, the ultrasound endoscope system 10 performs the polarization process on the ultrasound transducer on which the transmission of the ultrasonic wave for performing ultrasound diagnosis and the reception of the reflected wave are not performed. Therefore, even during the execution period of the ultrasound diagnosis, the frame rate is not reduced. As a result, since the reception sensitivities of the plurality of ultrasound transducers 48 can always be kept satisfactory without reducing the image quality of the ultrasound image, a high-quality ultrasound image can always be acquired.

In addition, since the ultrasound endoscope system 10 performs the transmission of the ultrasonic wave for performing the ultrasound diagnosis simultaneously with the polarization process using the existing transmission circuit 144, more specifically, the pulse generating circuit 158, it is possible to perform the polarization process during the execution period of the ultrasound diagnosis without significantly changing the existing circuit.

The total number of ultrasound transducers 48 and the number of opening channels may be changed to any number. For example, in a case where the number of opening channels is the same as the total number of the ultrasound transducers 48, the number of the driving target transducers 48 that perform the transmission of the ultrasonic waves for performing the ultrasound diagnosis and reception of the reflected waves needs to be smaller than the total number of the ultrasound transducers 48. In this case, it is possible to simultaneously perform the polarization process on the ultrasound transducer 48 to be polarized, which does not perform the transmission of the ultrasonic wave and the reception of the reflected wave.

While the present invention has been described in detail, the present invention is not limited to the above-described embodiments, and various improvements and modifications may be made without departing from the gist of the present invention.

### Explanation of References

10: ultrasound endoscope system
12: ultrasound endoscope
14: ultrasound processor device
16: endoscope processor device
18: light source device
20: monitor
21a: water supply tank
21b: suction pump
22: insertion part
24: operating part
26: universal cord
28a: water/air supply button
28b: suction button
29: angle knob
30: treatment tool insertion port
32a: ultrasound connector
32b: endoscope connector
32c: light source connector
34a: air/water supply tube
34b: suction tube
36: ultrasound observation portion
38: endoscope observation portion
40: distal end portion
42: bendable portion
43: soft portion
44: treatment tool outlet port
45: treatment tool channel
46: ultrasound transducer unit
48: ultrasound transducer
50: ultrasound transducer array
50a, 50b, 50c, 50d, 50e, 50f, 50g, 50h, 51a, 51b, 51c, 51d: element group
54: backing material layer
56: coaxial cable
58: endoscope side memory
60: FPC
74: acoustic matching layer
76: acoustic lens
82: observation window
84: objective lens
86: solid-state imaging element
88: illumination window
90: cleaning nozzle
92: wiring cable
100: console
140: multiplexer
142: reception circuit
144: transmission circuit
146: A/D converter
148: ASIC
150: cine memory
151: memory controller
152: CPU
154: DSC
158: pulse generating circuit
160: phase matching device
162: B mode image generation unit
164: PW mode image generation unit
166: CF mode image generation unit

## Claims

1. An ultrasound endoscope system that acquires an ultrasound image and an endoscope image, the ultrasound endoscope system comprising:
an ultrasound endoscope including an ultrasound observation portion, which has an ultrasound transducer array in which a plurality of ultrasound transducers that simultaneously transmit and open are arranged,
which transmits an ultrasonic wave using the plurality of ultrasound transducers on one side of the plurality of ultrasound transducers, receives a reflected wave of the ultrasonic wave due to the transmission of the ultrasonic wave, and pauses the transmission of the ultrasonic waves by the plurality of ultrasound transducers on the other side of the plurality of ultrasound transducers; and
an ultrasound processor device including a transmission circuit, which transmits an ultrasound generating transmission signal consisting of diagnostic driving pulses to be applied to the plurality of ultrasound transducers on one side to generate the ultrasonic waves from the plurality of ultrasound transducers on one side and simultaneously transmits a polarization process transmission signal consisting of polarization driving pulse for performing a polarization process to the plurality of ultrasound transducers on the other side in which the transmission of the ultrasound generating transmission signal for acquiring the ultrasound image is paused, a reception circuit, which outputs a reception signal based on the reflected waves received by the plurality of ultrasound transducers on one side, and an ultrasound image generation unit, which generates the ultrasound image by imaging the reception signal to acquire the ultrasound image,
wherein the ultrasound processor device further includes a control circuit that transmits the ultrasound generating transmission signal to the plurality of ultrasound transducers on one side using the transmission circuit to cause the plurality of ultrasound transducers on one side to perform the transmission of the ultrasonic wave and the reception of the reflected wave, to acquire the ultrasound image, and that transmits the polarization process transmission signal to the plurality of ultrasound transducers on the other side using the transmission circuit to be subjected to the polarization process, and
the control circuit controls the transmission circuit such that the transmission of the ultrasound generating transmission signal for generating the ultrasound image and the transmission of the polarization process transmission signal for performing the polarization process are simultaneously performed on the plurality of ultrasound transducers on one side and the plurality of ultrasound transducers on the other side different from each other, and simultaneously performs transmission and reception of the ultrasonic wave and the polarization process on the plurality of ultrasound transducers different from each other.

2. The ultrasound endoscope system according to claim 1,
wherein a transmission waveform of the ultrasound generating transmission signal is different from a transmission waveform of the polarization process transmission signal.

3. The ultrasound endoscope system according to claim 2,
wherein at least one of a frequency, voltage, or wave number of the transmission waveform is different between the ultrasound generating transmission signal and the polarization process transmission signal.

4. The ultrasound endoscope system according to claim 1 or 2,
wherein the control circuit changes a simultaneous transmission opening number of the plurality of ultrasound transducers on the other side on which the polarization process is performed, in response to a change in a simultaneous transmission opening number of the plurality of ultrasound transducers on one side that perform the transmission and reception of the ultrasonic wave for generating the ultrasound image in accordance with a change in a focus position for observation.

5. The ultrasound endoscope system according to claim 1 or 2,
wherein, in a case of using the ultrasound transducer array in which the plurality of ultrasound transducers are arranged in a circumferential shape, a center of the plurality of ultrasound transducers on one side that perform the transmission and reception of the ultrasonic wave for generating the ultrasound image and a center of the plurality of ultrasound transducers on the other side that perform the transmission of the polarization process transmission signal for performing the polarization process are arranged at positions different from each other by 180 degrees.

6. The ultrasound endoscope system according to claim 1 or 2,
wherein the control circuit controls,
in a case where the transmission of the ultrasonic wave and the reception of the reflected wave are performed using the plurality of ultrasound transducers on one side, simultaneously, the plurality of ultrasound transducers on the other side to pause the transmission and reception of the ultrasonic wave and to be subjected to the polarization process, and
conversely, in a case where the transmission of the ultrasonic wave and the reception of the reflected wave are performed using the plurality of ultrasound transducers on the other side, simultaneously, the plurality of ultrasound transducers on one side to pause the transmission and reception of the ultrasonic wave and to be subjected to the polarization process.

7. An operation method of an ultrasound endoscope system that acquires an ultrasound image and an endoscope image,
the ultrasound endoscope system including an ultrasound endoscope including an ultrasound observation portion, which has an ultrasound transducer array in which a plurality of ultrasound transducers are arranged, and an ultrasound processor device including a transmission circuit, which transmits an ultrasound generating transmission signal to the plurality of ultrasound transducers on one side of the plurality of ultrasound transducers and simultaneously transmits a polarization process transmission signal to the plurality of ultrasound transducers on the other side in which the transmission of the ultrasound generating transmission signal for acquiring the ultrasound image is paused, a reception circuit, which outputs a reception signal based on reflected waves received by the plurality of ultrasound transducers on one side, and an ultrasound image generation unit, which generates the ultrasound image by imaging the reception signal, the operation method comprising:
a control step of controlling the transmission circuit to generate the ultrasound generating transmission signal consisting of diagnostic driving pulses to be applied to the plurality of ultrasound transducers on one side, which generate ultrasonic waves for acquiring the ultrasound image, and, for a polarization process, to generate the polarization process transmission signal consisting of polarization driving pulses applied to the plurality of ultrasound transducers on the other side in which the transmission of the ultrasound generating transmission signal is paused;
a generating step of transmitting the ultrasound generating transmission signal generated from the transmission circuit to the plurality of ultrasound transducers on one side, and applying the diagnostic driving pulses to the plurality of ultrasound transducers on one side to generate the ultrasonic waves;
a reception step of receiving the reflected waves of the ultrasonic waves via the plurality of ultrasound transducers on one side;
an output step of outputting the reception signal based on the reflected waves received by the plurality of ultrasound transducers, from the reception circuit;
a generation step of generating the ultrasound image via the ultrasound image generation unit receiving the reception signal and imaging the received reception signal; and
a polarization step of transmitting the polarization process transmission signal generated from the transmission circuit to the plurality of ultrasound transducers on the other side and applying the polarization driving pulses to the plurality of ultrasound transducers on the other side to perform the polarization process on the plurality of ultrasound transducers on the other side simultaneously while executing the generating step and the reception step.

8. The operation method of an ultrasound endoscope system according to claim 7,
wherein a transmission waveform of the ultrasound generating transmission signal is different from a transmission waveform of the polarization process transmission signal.

9. The operation method of an ultrasound endoscope system according to claim 8,
wherein at least one of a frequency, voltage, or wave number of the transmission waveform is different between the ultrasound generating transmission signal and the polarization process transmission signal.

10. The operation method of an ultrasound endoscope system according to any one of claims 7 to 9,
wherein a simultaneous transmission opening number of the plurality of ultrasound transducers on the other side on which the polarization process is performed is changed, in response to a change in a simultaneous transmission opening number of the plurality of ultrasound transducers on one side that perform transmission and reception of the ultrasonic wave for generating the ultrasound image in accordance with a change in a focus position for observation.

11. The operation method of an ultrasound endoscope system according to any one of claims 7 to 9,
wherein, in a case of using the ultrasound transducer array in which the plurality of ultrasound transducers are arranged in a circumferential shape, a center of the plurality of ultrasound transducers on one side that perform transmission and reception of the ultrasonic wave for generating the ultrasound image and a center of the plurality of ultrasound transducers on the other side that perform the transmission of the polarization process transmission signal for performing the polarization process are arranged at positions different from each other by 180 degrees.

12. The operation method of an ultrasound endoscope system according to any one of claims 7 to 9,
wherein, in the control step, in a case of being controlled to generate the ultrasound generating transmission signal to be transmitted to the plurality of ultrasound transducers on one side, a control is performed to generate the polarization process transmission signal to be transmitted to the plurality of ultrasound transducers on the other side,
the generating step and the reception step are performed on the plurality of ultrasound transducers on one side, and the polarization step is simultaneously performed on the plurality of ultrasound transducers on the other side,
conversely, in the control step, in a case of being controlled to generate the ultrasound generating transmission signal to be transmitted to the plurality of ultrasound transducers on the other side, a control is performed to generate the polarization process transmission signal to be transmitted to the plurality of ultrasound transducers on one side, and
the generating step and the reception step are performed on the plurality of ultrasound transducers on the other side, and the polarization step is simultaneously performed on the plurality of ultrasound transducers on one side.
